# EUROPÄISCHE PATENTSCHRIFT

(11) **EP 0 298 918 B1**
(45) Veröffentlichungstag und Bekanntmachung des Hinweises auf die Patenterteilung: **05.09.2001**
(21) Anmeldenummer: 88810452.8
(22) Anmeldetag: 01.07.1988
(51) Int. Cl.: C12N 15/00, A01N 63/00, A01N 65/00, C12N 1/20, A01H 1/00, C12N 5/00

(54) **Induzierbare Virusresistenz bei Pflanzen**
Inducible virus resistance in plants
Résistance inductible contre des virus dans des plantes

(30) Priorität: 10.07.1987 CH 264587
(43) Veröffentlichungstag der Anmeldung: 11.01.1989
(73) Patentinhaber: Syngenta Participations AG, 4058 Basel (CH)
(72) Erfinder: Hohn, Thomas, Dr., CH-4103 Bottmingen (CH); Bonneville, Jean-Marc, Dr., CH-4057 Basel (CH); Fütterer, Johannes, Dr., CH-4051 Basel (CH); Gordon, Karl, Dr., D-7858 Weil am Rhein (DE); Sanfaçon, Hélène, Dr., CH-4057 Basel (CH)

(56) Entgegenhaltungen:
- EP-A- 0 194 809
- EP-A- 0 223 452
- EP-A- 0 242 016
- EP-A- 0 278 667
- NATO ASI SERIES. SERIES A, LIFE SCIENCES, Band 140 (Plant. Mol. Biol.), ed. Diter von Wettstein/Nam-Hai Chua. PROCEEDINGS NATO ADVANCED STUDY INSTITUTE ON PLANT MOLECULAR BIOLOGY, Carlsberg, 10.-19. Juni 1987, Seite 630, Plenum Press, New York, US; J.M. BONNEVILLE et al.: "Post-transcriptional regulation: Cauliflower mosaic virus gene I expression"
- MOLECULAR AND CELLULAR BIOLOGY, Band 7, Nr. 1, Januar 1987, Seiten 335-341, American Society for Microbiology; M.A. LAWTON et al.: "Transcriptional activation of plant defense genes by fungal elicitor, wounding, and infection"
- NATURE, Band 321, 22. Mai 1986, Seiten 446-449; D.C. BAULCOMBE et al.: "Expression of biologically active viral satellite RNA from the nuclear genome of transformed plants"
- THE EMBO JOURNAL, Band 6, Nr. 2, Februar 1987, Seiten 303-306, IRL Press, Eynsham, Oxford, GB; J.J. SANCHEZ-SERRANO et al.: "Wound-induced expression of a potato proteinase inhibitor II gene in transgenic tobacco plants"
- THE EMBO JOURNAL, Band 6, Nr. 7, Juli 1987, Seiten 1845-1851, IRL Press Ltd, Oxford, GB; L.S. LOESCH-FRIES et al.: "Expression of alfalfa mosaic virus RNA 4 in transgenic plants confers virus resistance"

## Beschreibung

Gegenstand der vorliegenden Erfindung ist ein Verfahren, welches auf der Grundlage gentechnologischer Methoden die Ausbildung einer induzierbaren Virusresistenz bei Pflanzen ermöglicht. Weiterhin bezieht sich die vorliegende Erfindung auf die Verwendung dieses Verfahrens zur Herstellung virusresistenter Pflanzen sowie auf die nach diesem Verfahren erhältlichen, mit einer induzierbaren Virusresistenz ausgestatteten Pflanzen selbst und auf deren Nachkommen.

Ein Grossteil der heute bekannten Krankheiten unserer wichtigsten Kulturpflanzen, die immer wieder zu beträchtlichen Ernte- und Ertragseinbussen im Obst-, Gemüse- und Getreideanbau führen, werden durch Pflanzenviren verursacht. Die Uebertragung dieser pflanzenpathogenen Viren erfolgt dabei in erster Linie durch saugende oder beissende phytophage Insekten und Nematoden, die als Vektoren fungieren.

Man ist daher im Kulturpflanzenbau schon sehr lange bemüht, geeignete Verfahren zum Schutz der Kulturpflanzen vor einem Virusbefall zu entwickeln.

Eine dieser Massnahmen, die jedoch nur bedingt wirksam und zudem mit erheblichen Risiken verbunden ist, wurde unter dem Begriff der 'cross protection' bekannt. Es handelt sich dabei um die artifizielle Infektion gesunder Kulturen mit abgeschwächten Stämmen pathogener Viren, wodurch eine Zweitinfektion mit dem eigentlichen Pathogen verhindert werden soll. Dieses Verfahren wurde z.B. zur Verringerung der Ertragsverluste im Tomaten-, Kartoffel- und Citrusanbau angewendet, die durch pathogene Viren, wie den Tomaten Mosaik Virus, das Kartoffel Spindelknollen Viroid und den Citrus Tristeza Virus, hervorgerufen werden. (Broadbent L, Ann. Rev. Phytopathol., 14: 75, 1976; Fernow KH, Phytopathology, 57: 1347, 1967; Costa AS and Muller GW, Plant Dis., 64: 538, 1980).

Die Anwendung dieses Verfahrens zum Schutz gesunder Kulturen gegen eine Virusinfektion bringt jedoch zahlreiche Nachteile und Gefahren mit sich.

So kann beispielsweise der für die artifizielle Infektion verwendete abgeschwächte Virusstamm aufgrund einer Mutation in einen hochvirulenten Stamm übergehen und somit selbst den Ausbruch einer Krankheit verursachen.

Weiterhin ist es möglich, dass ein bestimmter Virusstamm zwar zum Schutz einer bestimmten Pflanzenspezies durchaus geeignet ist, bei einer anderen Spezies dagegen als Pathogen wirkt und schwere Schädigungen verursacht.

Darüberhinaus sind synergistische Wirkungen zweier oder mehrerer Virus-Arten beschrieben, die zu neuen bisher unbekannten Krankheitssymptomen führen (Garces-Orejuela C and Pound GS, Phytopathology, 57: 232, 1957).

Mit der Einführung gentechnologischer Arbeitsmethoden auch in die Pflanzengenetik und den sich daraus ergebenden Möglichkeiten zur Einschleusung fremden genetischen Materials in Pflanzen, wurden neue Verfahren entwickelt, die auf der Expression nur einzelner Virusgene in den entsprechenden Pflanzen beruhen, anstelle der bisher praktizierten Infektion mit dem gesamten Virusgenom.

Bei den erwähnten neuen Verfahren werden beispielsweise Hüllproteingene bestimmter Viren (Tabak Mosaik Virus, Alfalfa Mosaik Virus) mit Hilfe der rekombinanten DNA Technologie ins Pflanzengenom eingebaut (Bevan MW et al., EMBO J., 4(8): 1921-1926, 1985; Abel PP et al., Science, 232: 738-743, 1986).

Bei der Expression des integrierten Hüllproteingens in der Pflanze kann durch das produzierte Hüllprotein eine gewisse Schutzwirkung erreicht werden, die die Pflanze vor einer Infektion mit dem entsprechenden Virus schützt bzw. die auftretenden Krankheitssymptome abschwächt. Die genauen mechanistischen Hintergründe für diese Schutzwirkung durch das Hüllprotein sind bisher weitgehend unbekannt.

Auch die Expression weiterer Virusgene kann zu einer Schutzwirkung vor einer Infektion mit dem betreffenden Virus führen. So kommt es beispielsweise in transgenen Pflanzen, die Gegensinn ('antisense') RNA von Teilen der entsprechenden Viren synthetisieren, zu einer Hemmung der Virus-expression. Auch die Expression von Satelliten-RNA in transgenen Pflanzen kann zu einer Schutzwirkung führen, vermutlich aufgrund einer Konkurrenz mit dem infizierenden Virus um die Replikase (Baulcombe DC et al., Nature, 321: 446-449, 1986).

EP A-223 452 beschreibt schliesslich, dass die Expression viraler Hüll-proteine transgenen Pflanzen eine Resistenz gegen Virusinfektionen verleiht.

Diese Verfahren gewähren zwar einen gewissen Schutz gegenüber einer Virusinfektion und/oder deren Folgen, sie haben aber ebenfalls entscheidende Nachteile:
a) Bei hohen Inokulationskonzentrationen des Virus kann der Schutzfaktor austitriert werden, so dass eine Schutzfunktion nicht mehr gewährleistet ist.
b) der Schutzfaktor selbst kann die Ursache für die Auslösung von krankheitsähnlichen Zuständen bei der den Schutzfaktor synthetisierenden Pflanze sein.
   So ist beispielsweise das Virushüllprotein in der Lage, in biologische Membranen einzudringen (de Zoeten GA and Gaard G, Virus Research, 1: 713-725, 1984) und diese zu schädigen. Dies kann in der Folge zu Funktionsstörungen innerhalb der Zelle führen, insbesondere bei Chloroplasten und Mitochondrien.
c) Die Schutzfaktorgene werden ständig (konstitutiv) exprimiert, auch wenn keine Virusinfektion vorliegt, was zu einer deutlichen Verschlechterung der Energiebilanz der betreffenden Pflanzen führt.

Ziel der vorliegenden Erfindung ist es daher, zur Ueberwindung der oben genannten Nachteile ein System zu entwickeln, bei dem der Schutzfaktor von der transformierten Pflanze nicht mehr konstitutiv produziert wird, sondern bei dem die Synthese des besagten Schutzfaktors je nach Bedarf in der produzierenden Pflanze an- oder abgeschaltet werden kann.

Bonnville et al (NATO ASI Series, Aseries A, Life Sciences, Band 140, Proceedings NATO advanced study Institute on plant molecular biology, Carlsberg, 10.-19. Juni 1987, Seite 630, Plenum Press, NY) berichten über Cotransfektionsexperimente eines dicistronischen Konstrukts, in welchem ein Reportergen mit dem CaMV ORFI verknüpft ist, und viraler CaMV DNA. Verstärkte Expression des Reportergens wurde beobachtet.

Diese Aufgabe konnte jetzt im Rahmen der vorliegenden Erfindung überraschenderweise mit einfachen Massnahmen gelöst werden, indem ein System zur Verfügung gestellt wird, welches die Induktion von Genen auf der Grundlage viruseigener Kontrollmechanismen ermöglicht.

Ein Gegenstand der vorliegenden Erfindung betrifft somit ein Verfahren zum Schutz von Pflanzen vor einer Virusinfektion und/oder deren Folgen, das sich dadurch kennzeichnet, dass man in besagte Pflanzen ein induzierbares Schutzfaktorgen einbringt, das bei Bedarf, d.h. bei einer beginnenden Infektion mit einem Virus, exprimiert wird und die Produktion des Schutzfaktors steuert.

Ein weiterer Gegenstand der vorliegenden Erfindung betrifft ein Verfahren zum Schutz von Pflanzen vor einer Infektion mit pflanzenpathogenen Viren, das sich dadurch kennzeichnet, dass man in besagte Pflanzen ein induzierbares Schutzfaktorgen einbringt, welches mit virusspezifischen Kontrollelementen sowie mit in pflanzlichen Zellen aktiven Expressionssignalen in operabler Weise verknüpft ist, so dass die Expression besagten Schutzfaktorgens und damit die Produktion des Schutzfaktors durch den infizierenden Virus selbst kontrolliert wird.

Darüber hinaus betrifft die vorliegende Erfindung rekombinante DNA Moleküle, die eine induzierbare Virusresistenz auf Pflanzen übertragen, wobei die infizierenden Viren durch einen von der Pflanze synthetisierten Schutzfaktor neutralisiert werden.

Insbesondere betrifft die vorliegende Erfindung transgene virusresistente Pflanzen sowie lebensfähiger Teile virusresistenter transgener Pflanzen die mit einem rekombinanten DNA Molekül transformiert worden sind, welches für einen induzierbaren Schutzfaktor kodiert, der für die Neutralisation des infizierenden Virus verantwortlich ist sowie Mutanten und Varianten besagter transgener Pflanzen.

Unter einer "virusresistenten" Pflanze ist im Rahmen der vorliegenden Erfindung definitionsgemäss eine Pflanze zu verstehen, die bei einer normalerweise wirksamen Viruskonzentration überlebt und vorzugsweise normales Wachstum zeigt.

Die vorliegende Erfindung betrifft weiterhin Pflanzen, welche aus transformierten Pflanzenzellen, die besagtes rekombinantes DNA Molekül enthalten, regeneriert werden sowie deren Samen, darüberhinaus die Nachkommen von Pflanzen, die aus besagten transgenen Pflanzenzellen regeneriert worden sind sowie Mutanten und Varianten davon.

Unter Mutanten und Varianten transgener Pflanzen sind erfindungsgemäss solche Pflanzen zu verstehen, die noch die charakteristischen Eigenschaften der Ausgangspflanze besitzen, welche diese aufgrund der Transformation mit einem erfindungsgemässen r-DNA Molekül erhalten hat.

Ebenso umfasst von der vorliegenden Erfindung sind alle Kreuzungs- und Fusionsprodukte mit dem zuvor erwähnten transfermierten Pflanzenmaterial.

Weiterhin betrifft die Erfindung Verfahren zur Herstellung virusresistenter transgener Pflanzen, welches sich dadurch kennzeichnet, dass man eine Pflanze oder lebensfähige Teile davon mit einem rekombinanten DNA Molekül transformiert.

Unter lebensfähigen Teilen von Pflanzen sollen definitionsgemäss, ohne dass dies eine Limitierung des Erfindungsgegenstandes darstellt, z.B. pflanzliche Protoplasten, Zellen, Zellaggregate, Kallus, Samen, Pollen, Eizellen, Zygoten oder Embryonen verstanden werden.

Die vorliegende Erfindung umfasst ebenso chimäre genetische Konstruktionen, die ein Schutzfaktor-Gen enthalten, sowie Verfahren zu ihrer Herstellung, weiterhin Klonierungsvehikel und Wirtsorganismen sowie Methoden zur Uebertragung einer induzierbaren Virusresistenz auf Pflanzen.

Ein weiterer Gegenstand der vorliegenden Erfindung betrifft die Verwendung besagter rekombinanter DNA-Moleküle zur "Immunisierung" von Pflanzen gegen einen unerwünschten Virusbefall.

In der folgenden Beschreibung werden eine Reihe von Ausdrücken verwendet, die in der rekombinanten DNA Technologie, sowie in der Pflanzengenetik gebräuchlich sind.

Um ein klares und einheitliches Verständnis der Beschreibung und der Ansprüche sowie des Umfangs, der den besagten Ausdrücken zukommen soll, zu gewährleisten, werden die folgenden Definitionen aufgestellt:
Heterologe(s) Gen(e) oder DNA: Eine DNA Sequenz, die für ein spezifisches Produkt, Produkte oder eine biologische Funktion kodiert und die von einer anderen Spezies stammt als derjenigen, in welche das besagte Gen eingeschleust wird; besagte DNA Sequenz wird auch als Fremdgen oder Fremd-DNA bezeichnet.
Homologe(s) Gen(e) oder DNA: Eine DNA Sequenz, die für ein spezifisches Produkt, Produkte oder eine biologische Funktion kodiert und die aus der gleichen Spezies stammt, in welche das besagte Gen eingeschleust wird.
Synthetische(s) Gen(e) oder DNA: Eine DNA-Sequenz, die für ein spezifisches Produkt, Produkte oder eine biologische Funktion kodiert und die auf synthetischem Wege hergestellt sind.
Pflanzen-Promotor: Eine Kontrollsequenz der DNA Expression, die die Transkription jeder beliebigen homologen oder heterologen DNA Gensequenz in einer Pflanze gewährleistet, sofern besagte Gensequenz in operabler Weise mit einem solchen Promotor verknüpft ist.
Ueberproduzierender Pflanzen-Promotor (OPP): Pflanzen-Promotor, der in der Lage ist, in einer transgenen Pflanzenzelle die Expression einer jeden in operabler Weise verknüpften funktionalen Gensequenz(en) in einem Ausmass (gemessen in Form der RNA oder der Polypeptidmenge) zu bewirken, das deutlich höher liegt, als dies natürlicherweise in Wirtszellen, die nicht mit besagtem OPP transformiert sind, beobachtet wird.
Pflanze: Jedes photosynthetisch aktive Mitglied aus dem Reich Planta, das durch einen membranumhüllten Nukleus, ein in Form von Chromosomen organisiertes genetisches Material, membranumhüllte cytoplasmatische Organelle und der Fähigkeit zur Durchführung einer Meiose charakterisiert ist.
Pflanzen-Zelle: Strukturelle und physiologische Einheit der Pflanze, bestehend aus einem Protoplasten und einer Zellwand.
Pflanzengewebe: Eine Gruppe von Pflanzenzellen, die in Form einer strukturellen und funktionellen Einheit organisiert sind.
Pflanzenorgan: Ein abgegrenzter und deutlich sichtbar differenzierter Teil einer Pflanze, wie beispielsweise Wurzel, Stamm, Blatt oder Embryo.
Schutzfaktor: Ein- oder Mehrkomponenten-System, welches innerhalb der Pflanze auf eine Weise mit infizierenden Viren direkt oder indirekt interagiert, dass eine Schutzwirkung vor einer Virusinfektion und/oder deren Folgen gewährleistet ist.
Schutzfaktorgen/e: Nucleotidsequenz(en), die für oben definierten Schutzfaktor kodieren.
Virusspezifische Kontrollelemente: Regionen am Virusgenom, die mit einem Virusprodukt in einer Weise interagieren, dass die Replikation, Transkription, Translation oder andere posttranskriptionale Prozesse (in "cis") beeinflusst werden.
DNA-Expressions-Vektor: Klonierungs-Vehikel, wie z.B. ein Plasmid oder ein Bakteriophage, das alle Signal-Sequenzen enthält, die für die Expession einer inserierten DNA in einer geeigneten Wirtszelle notwendig sind.
DNA-Transfer-Vektor: Transfer-Vehikel, wie z.B. ein Ti-Plasmid oder ein Virus, das die Einschleusung von genetischem Material in eine geeignete Wirtszelle ermöglicht.

Im folgenden soll eine kurze Beschreibung der Abbildungen gegeben werden:
- Abbildung 1: zeigt die Konstruktion von Plasmid pHS1, die in Beispiel 1.1 ausführlich beschrieben ist.
- Abbildung 2A-C: zeigt die verschiedenen Möglichkeiten der Transaktivierung bei CaMV.

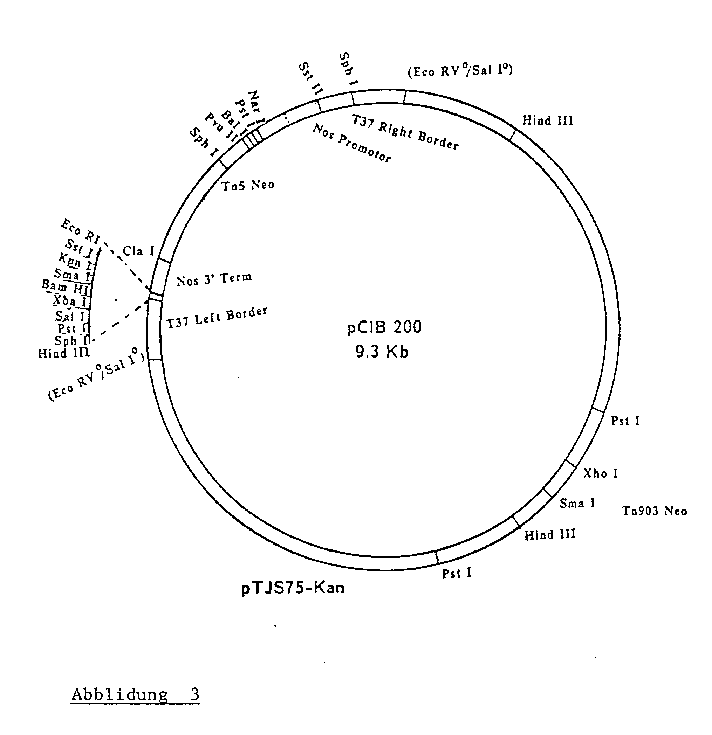

- Abbildung 3: zeigt die Restriktionskarte von Plasmid pCIB200.
- Abbildung 4: zeigt die Restriktionskarte von Plasmid V374.

Die vorliegende Erfindung betrifft in erster Linie rekombinante DNA-Moleküle, die in der Lage sind eine virusinduzierte Genexpression auf Pflanzen zu übertragen und das die folgenden Bestandteile aufweist:
(a) eine cis-aktive, virusspezifische Kontrollregion, die über einen auf der Transkriptions- oder Post-transkriptionsebene wirksamen Transaktivierungsmechanismus reguliert wird; und
(b) in funktioneller Verknüpfung damit eine DNA Sequenz, welche einen antiviral oder zelltoxisch wirkenden Schutzfaktor kodiert, wobei besagte DNA Sequenz gegebenenfalls zusätzlich mit in der pflanzlichen Zelle wirksamen Expressionssignalen operabel verknüpft wird.
Die Virusresistenz der Pflanzen wird dabei durch sogenannte Schutzfaktoren vermittelt, die von der transgenen Pflanze selbst produziert werden und die durch eine spezifische Interaktion mit dem infizierenden Pflanzenvirus dessen Reproduktionsfähigkeit in einem Masse beeinträchtigen, dass ein Ausbruch einer Infektion verhindert wird und/oder deren Folgen gemindert werden.

Man unterscheidet dabei grundsätzlich zwei Kategorien von Schutzfaktoren. Zum einen handelt es sich um antivirale Substanzen, die direkt am Virus angreifen und so dessen Reproduktionsfähigkeit negativ beeinflussen, z.B. durch Hemmung von Enzymen, die für den Entwicklungszyklus des Virus essentiell sind.

Eine zweite Kategorie betrifft zelltoxische Substanzen, welche die von den Viren befallenen Zellen vergiften und/oder abtöten und somit den Infektionsherd vom übrigen gesunden Gewebe isolieren. In diesem Fall wird also die Vermehrungsfähigkeit: des Virus indirekt durch die künstliche Auslösung einer "hypersensitiven Reaktion" eingeschränkt.

Im Rahmen der vorliegenden Erfindung sind daher unter Schutzfaktoren Ein-oder Mehrkomponenten-Systeme zu verstehen, die innerhalb der Pflanze direkt oder indirekt mit den infizierenden Viren interagieren, so dass eine Schutzwirkung vor einer Virusinfektion und/oder deren Folgen gewährleistet ist.

Beispiele solcher Schutzfaktoren, die aber keine Limitierung des Erfindungsgegenstandes darstellen, sind:
1) Antivirale Substanzen, wie z.B. Antivirus Antikörper, P_{R}-Proteine (Ahl P et al., Plant Mol. Biol., 4: 37, 1985), virusspezifische Protease-inhibitoren (Jamet E und Fritig B, Plant Mol. Biol., 6: 69-80, 1986) Proteasen, virusspezifische Polymerase-Inhibitoren, und Interferonähnliche Proteine.
2) Zelltoxische Substanzen, wie z.B. Toxine, aktive Untereinheiten von Toxinen, P_{R}-Proteine, sowie andere Proteine, die natürlicherweise Hypersensitivität bewirken, wie die β-Glucanase (Mohnen D. et al., EMBO J., 4: 1631-1635, 1985).

Von besonderer Bedeutung im Zusammenhang mit der vorliegenden Erfindung sind die Ribosomen inaktivierenden Proteine [RIPS] (Lord et al., Oxford Sur. of Plant Mol. Cell Biol., 1: 85-101, 1984) die eine irreversible Hemmung der Ribosomen verursachen und zu einer sorfortigen Einstellung der Proteinsynthese führen.

Beispiele solcher Ribosomen-inaktivierender Proteine sind das Abrin (aus Abrus precatorius Samen) (Olsnes et al., (In: Cohen P. and Van Heyninger S. (Ed.) Molecular Actions of Toxins and Viruses, Biomedical Press pp. 51-105, 1982) Modeccin (aus Modecca digitata Wurzeln) (Stirpe et al., FEBS Lett. , 85: 65-67, 1978), Viscumin (aus Viscum album Blättern) (Stirpe et al., Biochem. J., 190: 843-845, 1980; Olsnes et al., J. Biol. Chem., 257: 13263-13270, 1982) sowie Ricin (aus Ricinus communis) (Olsnes and Pihl, In: Cuatrecasas P. (Ed.) The Specificity of Action of Animal, Bacterial and Plant Toxins. Chapman and Hall, pp. 129-173, 1976). Ebenfalls in diese Gruppe gehört das 'Pokeweed'-Toxin (Owens et al., Virology, 56: 390, 1973).
All diese genannten Verbindungen weisen sowohl eine grosse strukturelle als auch funktionelle Aehnlichkeit auf.

Als besonders vorteilhaft erweist es sich, wenn die Induktion des Schutzfaktorgens und damit der Schutzfaktor-Produktion durch den infizierenden Virus selbst erfolgt. Auf diese Weise bleibt die Synthese des Schutzfaktors auf die Fälle beschränkt, in denen eine Virusinfektion stattfindet und der Schutzfaktor tatsächlich benötigt wird, um der Pflanze den entsprechenden Schutz zu gewähren.

Wird die Pflanze von einem entsprechenden Virus befallen, so wird die virusspezifische Kontrollregion angeschaltet und damit das Schutzfaktorgen exprimiert. In der Folge kommt es zur Synthese des Schutzfaktors, der den infizierenden Virus neutralisiert. Je mehr infizierender Virus vorhanden ist, desto mehr Schutzfaktor wird produziert und desto mehr Virus wird neutralisiert.

Damit ist es im Rahmen der vorliegenden Erfindung erstmals gelungen, einen in sich geschlossenen Kontrollkreis aufzubauen, der sich selbst reguliert und der Pflanze einen optimalen Schutz vor einer Virusinfektion gewährleistet.

Dies führt in der Folge dazu, dass von der Pflanze nicht unnötig Energie aufgewendet werden muss, um einen dauerhaften Schutz vor einer Virusinfektion aufrecht zu erhalten, die andererseits dringend für das Wachstum und die Fruchtbildung benötigt wird. Dies kann erfindungsgemäss beispielsweise dadurch erreicht werden, dass man das Schutzfaktorgen mit virusspezifischen Kontrollelementen verknüpft und damit deren Expression unter die Kontrolle besagter Kontrollelemente stellt.

Ein weiterer Gegenstand der vorliegenden Erfindung betrifft daher ein Verfahren zum Schutz von Pflanzen vor einer Virusinfektion und deren Folgen auf der Grundlage viruseigener Kon-trollmechanismen, dadurch gekennzeichnet, dass man
(a) eine DNA Sequenz, welche einen antiviral oder zelltoxisch wirkenden Schutzfaktor kodiert, mit einer cis-aktiven virusspezifischen Kontrollregion, die über einen auf der Transkriptions- oder Posttranskriptions bzw. -translationsebene wirksamen Transaktivierungsmechanismus reguliert wird, sowie gegebenenfalls mit in der pflanzlichen Zelle wirksamen Expressionssignalen operabel verknüpft; und
(b) das chimäre Konstrukt gemäss (a) in die zu schützende Pflanze einbringt, sodass es nach Infektion der Pflanze mit viraler DNA zu einer virusinduzierten Expression des Schutzfaktorgens kommt

Es ist bekannt, dass eine Vielzahl von Viren spezifische Kontrollmechanismen entwickelt hat, die zur Koordination der einzelnen Virusfunktionen essentiell sind.

Zu den genannten virusspezifischen Kontrollfunktionen gehören beispielsweise:
a) die Transaktivierung von Virusgenen auf der Transkriptions- und der Post-Transkriptionsebene
b) Herstellung subgenomischer RNA durch viruskodierte Replikasen
c) Aktivierung von Proteinen durch Prozessierung, Phosphorylierung und andere Modifikationen.

Das Phänomen der viralen Transaktivierung ist bisher fast ausschliesslich bei bakteriellen und tierischen Systemen (Calender, Biotechnology, 4: 1074, 1986) beobachtet worden.

Es existieren grundsätzlich verschiedene Ebenen auf denen ein Transaktivierungsmechanismus angreifen kann.
Der einfachste und offensichtlichste Weg ist die Transaktivierung der Transkription.
Dieser Typ der Transaktivierung ist beispielsweise als einer der Hauptwirkungsmechanismen für das humane HTLV-System beschrieben worden.
Dabei reguliert der Transaktivator die Transkription durch Reaktion mit dem viralen LTR ('Long Terminal Repeat') (Muesing MA et al., Cell, 48: 691-701, 1987).

Bestimmte Leseraster dieses Virus (HIV-LTR), wie z.B. das tat-Gen kodieren für Transaktivatoren, die die Expression der Virusgene gag-pol-env ermöglichen. Bei dem tat Genprodukt handelt es sich um ein Polypeptid vom MG 15 000, das in seinem Aufbau und seinen Eigenschaften an Nukleinsäure-Bindungsproteine erinnert, wie z.B. den Transkriptionsfaktor VIII A von Xenopus laevis u.a.

Als möglicher Mechanismus für die Transaktivierung wird daher eine kooperative Bindung eines Regulators an eine Erkennungsstelle in Betracht gezogen. Eine solche Erkennungsstelle existiert beispielsweise im Bereich der RNA Start-Region.

Auf der posttranskriptionalen Ebene dagegen stehen eine Vielzahl von verschiedenen Transaktivierungsmechanismen zur Verfügung, die an sehr unterschiedlichen Stellen angreifen können. Die Möglichkeiten reichen dabei von der Kontrolle der mRNA-Verlängerung (Grayhack et al., Cell, 42: 259, 1985; Yi-Kao et al., Nature, 330: 489 1987), über die Kontrolle des mRNA Transports vom Nukleus ins Cytoplasma, des mRNA Spleissens, der mRNA Polyadenylierung (Derse, J. Virol., 62, 1115, 1988) der Translations-Initation (Jay et al., Proc. Natl. Acad. Sci, USA, 78: 2927, 1981) bis hin zur differenzierten Verwendung verschiedener ORF's ('Open Reading Frame') auf der RNA.

Eine dritte Ebene der möglichen Transaktivierungsmechanismen betrifft die posttranslationale Modifikation des Protein-Produkts in Form von Phosphorylierungen oder einem Protein-Processing.

Im Falle des Cauliflower Mosaik Virus (CaMV) sind alle drei der zuvor genannten Transaktivierungsebenen von Relevanz.

CaMV besitzt ein doppelsträngiges (ds) zirkuläres DNA-Genom, dessen Replikation vermutlich über einen reversen Transkriptionsmechanismus erfolgt. Dabei werden zwei RNA Zwischenprodukte gebildet, eine 35 S RNA und eine 19 S RNA, die zwei Operons auf dem CaMV Genom entsprechen. Vom 19 S Promoter wird eine subgenomische RNA (19 S RNA Transkript) transkribiert, die das CaMV Protein VI kodiert, welches die Hauptmasse der virusspezifischen Einschlusskörperchen bildet. Die 35 S RNA besteht aus einer 600 Nukleotide umfassenden Leader-Region gefolgt (in Reihenfolge) von den Leserastern VII, I, II, III, IV, V und VI. Es wird angenommen, dass alle diese Leseraster, mit Ausnahme des Leserasters VI, von der 35 S RNA translatiert werden. (Pfeiffer P und Hohn T, Cell, 33: 781 - 789, 1983; Kridl JC and Goodman RM, Bio Essays, 4: 4 - 8, 1986).

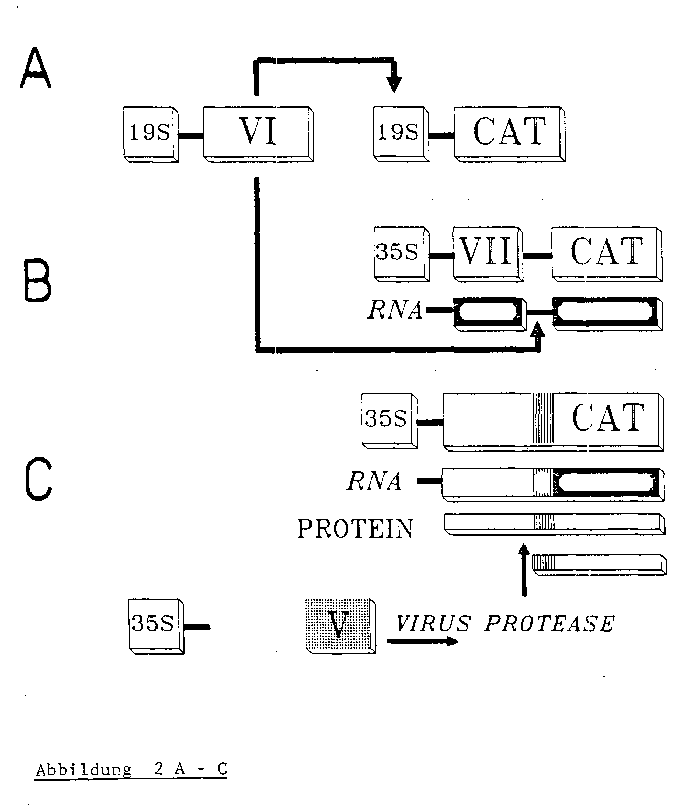

Im Rahmen der vorliegenden Erfindung konnten die folgenden Kontrollmechanismen für das CaMV-System verifiziert werden:
1) Der 19 S Promoter wird durch das Proteinprodukt des 19 S Operon in Form einer Autokatalyse aktiviert (siehe Abb. 2A).
2) Darüberhinaus wird auch die Verwendung der polycistronischen RNA durch das besagte Proteinprodukt des 19 S Operons reguliert (siehe Abb. 2B).
3) Die Freisetzung eines spezifischen Proteinproduktes erfolgt aus einem Polyprotein durch die Aktivität viraler Proteasen (Abb. 2C).

Bei bestimmten Strang-positiven(+) Viren findet man eine Variation dieser viralen transskriptionellen Transaktivierung. In diesen Fällen erfolgt eine Produktion subgenomischer Virus (messenger) RNA ausgehend von minus-Strang RNA, katalysiert durch virus-spezifische Polymerasen.

Diese Sonderform einer transkriptionalen Transaktivierung ist beispielsweise beim Brome Mosaik Virus (BMV) verwirklicht, der Pflanzen aus der Gruppe der Monokotyledonen insbesondere zahlreiche Gramineenarten, wie z.B. Mais, Gerte, Bromgras und andere Wildgräser, darüberhinaus aber auch verschiedene dikotyle Pflanzen befällt.

Das Genom von BMV ist aus insgesamt drei RNA Komponenten zusammengesetzt. Die Transkripte von zwei dieser Komponenten (RNA 1 und RNA 2) sind für die Replikation des viralen Genoms in der Pflanze essentiell. So ist beispielsweise die Replikation der dritten Komponente (RNA 3), einer dicistronischen RNA, die für ein 32 000 MG Protein und für das Hüllprotein kodiert sowie die Produktion subgenomischer Hüllprotein-mRNA von der Anwesenheit der RNA 1 und RNA 2 Transkripte abhängig (French et al., Science, 231: 1294-1297, 1986).

Als Mechanismus für die Herstellung subgenomischer mRNA zur Expression der auf der RNA 3 befindlichen Gene, dient vermutlich eine interne Initiation einer virusspezifischen Replikase, die sehr früh im Verlaufe des Infektionszyklus gebildet wird (evtl. kodiert durch genomische RNA 1 und/oder RNA 2) am minus(-) Strang der genomischen RNA 3 (Miller et al., Nature, 313: 68-70, 1985).

Darüberhinaus kann man davon ausgehen, dass sich die Kopienzahl genomischer Nukleinsäuren (DNA oder RNA) sehr stark erhöhen lässt, wenn diese mit viralen Replikationssignalen versehen werden und zwar aufgrund der Aktivität spezifischer viraler Polymerasen.

Alle diese genannten Systeme können in dem erfindungsgemässen Verfahren für die Konstruktion von Vektoren genutzt werden, die in der Lage sind eine induzierbare Virusresistenz auf pflanzliches Material zu übertragen

Besonders bevorzugt im Rahmen dieser Erfindung ist die Verwendung der virusspezifischen Kontrollelemente des Cauliflower Mosaik Virus (CaMV), was jedoch den Umfang der vorliegenden Erfindung in keiner Weise einschränkt, sondern lediglich dazu dient, das Funktionieren des erfindungsgemässen Verfahrens zu demonstrieren.

Ein weiterer Gegenstand der vorliegenden Erfindung bezieht sich somit auf die Herstellung hybrider Genkonstruktionen, die zusammengesetzt sind aus einer oder mehreren virusspezifischen Kontrollregionen sowie aus homologen oder heterologen DNA-Sequenzen, die für ein oder mehrere Schutzfaktoren X kodieren, unter Verwendung von Methoden und Verfahren, die auf dem Gebiet der rekombinanten DNA Technologie weitgehend bekannt und gebräuchlich sind, und die unter anderem in den folgenden Publikationen beschrieben sind:

"Molecular Cloning", Maniatis T, Fritsch EF und J Sambrook, Cold Spring Harbor Laboratory, 1982, und "Recombinant DNA Techniques", Rodriguez RL und RC Tait, Addison-Wesley Publishing Comp., London, Amsterdam, Don Mils. Ontario, Sydney, Tokyo, 1983.

Die für das Schutzfaktor-Gen kodierende Region, die gemäss der vorliegenden Erfindung verwendet wird, kann homologenen oder heterologenen Ursprungs sein, im Verhältnis zu der Pflanzenzelle oder der Pflanze, die transformiert werden soll. Es ist jedoch auf alle Fälle notwendig, dass die Gensequenz, die für den Schutzfaktor kodiert exprimiert wird und zur Produktion eines funktionstüchtigen Enzyms oder Polypeptids in der resultierenden Pflanzenzelle führt.

Unter DNA-Sequenzen heterologen Ursprungs sind erfindungsgemäss solche zu verstehen, die aus anderen Pflanzen-Spezies oder aus Organismen stammen, die einer anderen taxonomischen Einheit angehören, so z.B. aus Mikroben oder aus Säugern oder die synthetischen Ursprungs sind und die in der Lage sind in der jeweiligen gewünschten Pflanzenspezies die gewünschte Schutzfunktion gegen einen Virusbefall zu erreichen.

Die kodierende Region der hybriden Genkonstruktion kann auch einen Schutzfaktor kodieren, der sich von einem natürlich vorkommenden unterscheidet, der aber immer noch im wesentlichen die Schutzfunktion des natürlichen Schutzfaktors erfüllt. Solch eine kodierende Sequenz wird gewöhnlicherweise eine Variante einer natürlichen kodierenden Region sein. Unter einer "Variante" einer natürlichen DNA-Sequenz ist im Rahmen dieser Erfindung definitionsgemäss eine modifizierte Form einer natürlichen Sequenz zu verstehen, die aber noch dieselbe Funktion erfüllt. Die Variante kann eine Mutante oder eine synthetische DNA-Sequenz sein und ist im wesentlichen der entsprechenden natürlichen Sequenz homolog. Im Rahmen dieser Erfindung ist eine DNA-Sequenz einer zweiten DNA-Sequenz dann im wesentlichen homolog, wenn wenigstens 70 %, vorzugsweise wenigstens 80 %, insbesondere aber wenigstens 90 % der aktiven Abschnitte der DNA-Sequenz homolog sind. Gemäss vorliegender Definition des Begriffs "im wesentlichen homolog" werden zwei unterschiedliche Nukleotide in einer DNA-Sequenz einer kodierenden Region dann als homolog angesehen, wenn der Austausch des einen Nukleotids gegen das andere eine stille Mutation darstellt.

Die Erfindung umfasst somit jedes, eine Aminosäuresequenz kodierende hybride Gen, das die gewünschte Schutzfunktion vermittelt, welche die offenbarten und beanspruchten Erfordernisse erfüllt. Besonders bevorzugt ist eine Nukleotidsequenz, die im wesentlichen wenigstens dem Teil oder den Teilen der natürlichen Sequenz homolog ist, der (die) für die Schutzfunktion gegen einen Virusbefall verantwortlich ist (sind).

Die für den Schutzfaktor kodierende DNA-Sequenz kann ausschliesslich aus genomischer, aus cDNA bzw. aus synthetischer DNA konstruiert sein. Eine andere Möglichkeit besteht in der Konstruktion einer hybriden DNA-Sequenz bestehend sowohl aus cDNA als auch aus genomischer DNA und/oder synthetischer DNA. In diesem Fall kann die cDNA aus demselben Gen stammen wie die genomische DNA oder aber, sowohl die cDNA, wie auch die genomische DNA können aus verschiedenen Genen stammen. In jedem Fall können aber, sowohl die genomischen DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehreren Organismen, die mehr als einem Stamm, einer Varietät oder einer Spezies derselben Gattung angehören, oder aber von Organismen, die mehr als einer Gattung derselben oder einer anderen taxono-mischen Einheit (Reich) angehören, abstammen.

Die verschiedenen DNA-Sequenzabschnitte können mit Hilfe an sich bekannter Methoden miteinander zu einer vollständigen für den Schutzfaktor kodierenden DNA-Sequenz verknüpft sein. Geeignete Methoden schliessen beispielsweise die in vivo Rekombination von DNA-Sequenzen, die homologe Abschnitte aufweisen sowie die in vitro Verknüpfung von Restriktionsfragmenten mit ein.

Es werden somit eine Vielzahl von Ausführungsformen von dem breiten Konzept dieser Erfindung umfasst.

Eine dieser erfindungsgemässen Ausführungsformen ist durch eine chimäre Gen-Sequenz gekennzeichnet, enthaltend:
(a) eine oder mehrere Gen-Sequenzen, die für ein oder mehrere Schutzfaktor Polypeptide kodieren, welche nach erfolgter Expression des Gens in einer gegebenen Pflanzenzelle eine Schutzfunktion vor einer Virusinfektion und/oder deren Folgen bewirkt, und
(b) eine oder mehrere Gensequenzen, die für eine oder mehrere virus-spezifische Kontrollregionen kodieren, welche in operabler Weise mit der für den Schutzfaktor kodierenden Region verknüpft ist, und
(c) eine oder mehrere zusätzliche Gensequenzen, die in operabler Weise mit beiden Seiten der für den Schutzfaktor sowie gegebenenfalls für die zugehörigen virusspezifischen Kontrollelemente kodierenden Region verknüpft sind. Diese zusätzlichen Gensequenzen enthalten Promotor-und Terminator-Regionen sowie gegebenenfalls zusätzlich regulatorische Sequenzen der 3'- und 5'-nichttranslatierten Regionen. Die pflanzlichen regulatorischen Sequenzen können im Verhältnis zu der Wirtszelle heterolog oder homolog sein .

Die funktionelle Verknüpfung der für einen Schutzfaktor kodierenden Region mit einer virusspezifischen Kontrollregion kann erfindungsgemäss z.B. dadurch erreicht werden, dass man viruseigene kodierende DNA-Sequenzen, von denen bekannt ist, dass sie natürlicherweise im Verlaufe des Infektionszyklus durch spezifische Funktionen des intakten Virus kontrolliert werden, isoliert und den kodierenden Abschnitt durch ein Schutzfaktorgen ersetzt, und somit das Schutzfaktorgen unter virale Kontrolle bringt. Als Beispiele solcher kodierender viraler Regionen, die aber in keiner Weise als limitierende aufzufassen sind, können das Leseraster I und VI des CaMV-Genoms genannt werden, sowie die Hüllprotein-kodierende DNA-Sequenz auf der dicistronischen RNA 3 des BMV.

Jeder Promotor und jeder Terminator, der in der Lage ist, die Expression einer für einen Schutzfaktor kodierenden DNA-Sequenz zu bewirken, kann als Bestandteil der chimären Gensequenz verwendet werden. Beispiele geeigneter Promotoren und Terminatoren sind z.B. solche der Nopalin-Synthase-Gene (nos), der Octopin-Synthase-Gene (ocs) sowie der Cauliflower Mosaik Virus Gene (CaMV).

Ein wirksamer Vertreter eines Pflanzenpromotors, der Verwendung finden kann, ist ein überproduzierender Pflanzenpromotor. Diese Art von Promotoren sollte, sofern sie in operabler Weise mit der Gensequenz für das Schutzfaktorgen verknüpft ist, in der Lage sein, die Expression besagter Schutzfaktoren in einer Weise zu vermitteln, dass die transformierte Pflanze gegenüber einem Virusbefall geschützt ist.

Ueberproduzierende Pflanzenpromotoren, die im Rahmen der vorliegenden Erfindung zur Anwendung kommen können, schliessen den Promotor der kleinen Untereinheit (small subunit; ss) der Ribulose-1,5-bisphosphat-Carboxylase aus Sojabohnen [Berry-Lowe et al., J. Molecular and App. Gen., 1: 483-498 (1982)] sowie den Promotor des Chlorophyll-a/b-Bindungs-proteins ein. Diese beiden Promotoren sind dafür bekannt, dass sie in eukaryontischen Pflanzenzellen durch Licht induziert werden [siehe z.B. Genetic Engineering of Plants, an Agricultural Perspective, A. Cashmore, Plenum, New York 1983, Seite 29-38; Coruzzi G et al., The Journal Of Biological Chemistry, 258: 1399 (1983) und Dunsmuir P et al., Journal of Molecular and Applied Genetics, 2: 285 (1983)].

Durch die Verwendung starker Promotoren ist gewährleistet, dass auch in Abwesenheit des Virus ständig geringe Mengen an Schutzfaktor-Protein synthetisiert werden, sodass bei einer akuten Virusinfektion eine sehr schnelle erste Reaktion auf das Infektionsereignis möglich ist.

Eine im Rahmen der vorliegenden Erfindung bevorzugte Gen-Konstruktion ist zusammengesetzt aus dem CaMV 35-S Promoter, aus einer Leader Sequenz, dem CaMV Leseraster VII, einer CaMV Leseraster I / Schutzfaktorgen Fusion sowie einem Transkriptions-Terminator.

Ebenfalls bevorzugt im Rahmen dieser Erfindung ist eine Genkonstruktion, die aus dem CaMV 19S-Promoter, einer Schutzfaktor-kodierenden DNA-Sequenz im ORF VI des CaMV-Genoms sowie einer CaMV Terminationssequenz zusammengesetzt ist.

Die chimäre Gensequenz, die aus einer oder mehreren virusspezifischen Kontrollsequenzen sowie einem oder mehreren Schutzfaktorgenen besteht und die in operabler Weise mit in pflanzlichen Zellen aktiven Expressionssignalen verknüpft ist, kann in einen geeigneten Klonierungsvektor eingespleisst werden. Im allgemeinen verwendet man Plasmid- oder Virus-(Bakteriophage)-Vektoren mit Replikations- und Kontrollsequenzen, die von Spezies stammen, die mit der Wirtszelle kompatibel sind.

Der Klonierungsvektor trägt in der Regel einen Replikationsursprung, ausserdem spezifische Gene, die zu phänotypischen Selektionsmerkmalen in der transformierten Wirtszelle führen, insbesondere zu Resistenzen gegenüber Antibiotika oder gegenüber bestimmten Herbiziden. Die transformierten Vektoren können anhand dieser phänotypischen Marker nach einer Transformation in einer Wirtszelle selektiert werden.

Als Wirtszellen kommen im Rahmen dieser Erfindung Prokaryonten in Frage, einschliesslich bakterieller Wirte, wie z.B. A.tumefaciens, A. rhizogenes, E.coli, S.typhimurium und Serratia marcescens, sowie Cyanobakterien. Ferner können auch eukaryontische Wirte wie Hefen, mycelbildende Pilze und Pflanzenzellen im Rahmen dieser Erfindung verwendet werden.

Der Klonierungsvektor und die mit diesem Vektor transformierte Wirtszelle werden erfindungsgemäss dazu verwendet, die Kopienzahl des Vektors zu erhöhen. Mit einer erhöhten Kopienzahl ist es möglich, den Vektor, der das Schutzfaktor-Gen trägt, zu isolieren und z.B. für die Einschleusung der chimären Gensequenz in die pflanzliche Zelle zu verwenden.

Die Einschleusung von DNA in Wirtszellen kann mit Hilfe von an sich bekannten Verfahren durchgeführt werden. So können beispielsweise bakterielle Wirtszellen nach einer Behandlung mit Calciumchlorid transformiert werden. In Pflanzenzellen lässt sich DNA durch direkten Kontakt mit den aus den Zellen hergestellten Protoplasten einschleusen.

Eine andere Möglichkeit zur Einführung von DNA in Pflanzenzellen besteht darin, dass man die Zellen mit Viren oder mit Agrobacterium in Kontakt bringt. Dies kann durch Infektion sensitiver Pflanzenzellen oder durch Co-Cultivierung von Protoplasten, die sich aus Pflanzenzellen ableiten, bewerkstelligt werden.

Diese Verfahren werden im folgenden noch genau diskutiert.

Für die direkte Einschleusung von DNA in Pflanzenzellen stehen eine Reihe von Verfahren zur Verfügung. So kann das genetische Material, das in einem Vektor enthalten ist, beispielsweise mit Hilfe von Mikropipetten für den mechanischen Transfer der rekombinanten DNA direkt in die Pflanzenzellen mikroinjiziert werden. Das genetische Material kann ebenso nach einer Behandlung der Protoplasten mit Polyethylenglykol in diese eingebracht werden. [Paszkowski J et al., EMBO J., 3: 2717-22 (1984)].

Ein weiterer Gegenstand der vorliegenden Erfindung betrifft die Einschleusung des Schutzfaktor-Gens in die Pflanzenzelle mit Hilfe der Elektroporation [Shillito R et al., Biotechnology, 3: 1099-1103 (1985); Fromm M et al., Proc. Natl. Acad. Sci. USA, 82: 5824 (1985)].

Bei dieser Technik werden pflanzliche Protoplasten in der Gegenwart von Plasmiden, die das Schutzfaktor-Gen enthalten, einer Elektroporation unterzogen.

Elektrische Impulse hoher Feldstärke führen zu einer reversiblen Permeabilitätserhöhung von Biomembranen und ermöglichen damit die Einschleusung der Plasmide. Elektroporierte pflanzliche Protoplasten erneuern ihre Zellwand, teilen sich und bilden Kallusgewebe. Die Selektion der transformierten Pflanzenzellen mit dem exprimierten Schutzfaktor kann mit Hilfe der zuvor beschriebenen phänotypischen Marker erfolgen.

Das Cauliflower Mosaik Virus (CaMV) kann im Rahmen dieser Erfindung ebenso als Vektor für die Einschleusung des Schutzfaktor Gens in die Pflanze verwendet werden (Hohn et al., in "Molecular Biology of Plant Tumors", Academic Press, New York, 1982, Seite 549-560; Howell, US-Patent Patent No. 4,407,956).

Das gesamte virale DNA-Genom von CaMV wird dabei in ein bakterielles Elternplasmid integriert unter Ausbildung eines rekombinanten DNA Moleküls, das in Bakterien vermehrt werden kann. Nach erfolgter Klonierung wird das rekombinante Plasmid mit Hilfe von Restriktionsenzymen entweder zufällig oder an ganz spezifischen nicht essentiellen Stellen innerhalb des viralen Teils des rekombinanten Plasmids gespalten, z.B. innerhalb des Gens, das für die Uebertragbarkeit des Virus durch Blattläuse kodiert, um die Schutzfaktor-Gensequenz einbauen zu können.

Ein kleines Oligonucleotid, ein sog. Linker, der eine einzige, spezifische Restriktionserkennungsstelle besitzt, kann ebenfalls integriert werden. Das so modifizierte rekombinante Plasmid wird erneut kloniert und durch Einspleissen der Schutzfaktor-Gensequenz in eine nur einmal vorkommende Restriktionsstelle weiter modifiziert.

Der modifizierte virale Anteil des rekombinanten Plasmids wird dann aus dem bakteriellen Eltern-Plasmid ausgeschnitten und für die Inokulation der Pflanzenzellen oder der gesamten Pflanzen verwendet.

Eine andere Methode zur Einschleusung der erfindungsgemässen hybriden Genkonstruktion in die Zelle bedient sich der Infektion der Pflanzenzelle mit Agrobacterium tumefaciens oder Agrobacterium rhizogenes, welches mit dem Schutzfaktor-Gen in einer Weise transformiert ist, dass besagtes Schutzfaktor-Gen die Tumor-produzierenden Gene zwischen den T-DNA Grenzsequenzen ersetzt. Eine weitere Möglichkeit im Rahmen dieser Erfindung besteht in einer gemischten Infektion unter Verwendung von sowohl A. rhizogenes als auch transformiertem A. tumefaciens wie es von Petit et al. (Petit et al., Mol. Gen. Genet. 202: 388, 1986) für die Transformation von Karotten beschrieben worden ist. Das Mischungsverhältnis muss dabei so aufeinander abgestimmt werden, dass die durch die A. rhizogenes Transformation hervorgerufenen Würzelchenkolonien auch einen hohen Anteil an A. tumefaciens Ti-Plasmid enthalten. Dies kann erfindungsgemäss dadurch erreicht wreden, dass man A. rhizogenes und A. tumefaciens in einem Mischungsverhältnis von 1:1 bis 1:100, vorzugsweise aber von 1:10 in bekannter Weise gemeinsam auf das Pflanzenmaterial appliziert. Die transgenen Pflanzenzellen werden anschliessend unter geeigneten, dem Fachmann bekannten Kultivierungsbedingungen kultiviert, so dass sie Schösslinge und Wurzeln ausbilden und letztlich ganze Pflanzen resultieren.
Die Mischung der beiden Agrobacterien-Spezies erfolgt vorteilhafterweise erst kurz vor der eigentlichen Inokulation.

Die Schutzfaktor kodierenden Gensequenzen lassen sich beispielsweise mit Hilfe des Ti-Plasmids von Agrobacterium tumefaciens [DeCleene et al., Bot. Rev., 47: 147-194 (1981); Bot. Rev., 42: 389-466 (1976)] sowie des Ri-Plasmids von A. rhizogenes (M Tepfer und F Casse-Delbart, Microbiol. Sci., 4(1): 24-28, 1987) in geeignete Pflanzenzellen überführen. Das Ti-Plasmid wird im Verlaufe der Infektion durch Agrobacterium tumefaciens auf die Pflanze übertragen und stabil ins Pflanzengenom integriert. [Horsch et al., Science, 233: 496-498 (1984); Fraley et al., Proc. Natl. Acad. Sci. USA, 80: 4803 (1983)].

Für Pflanzen, deren Zellen für eine Infektion mit Agrobacterium nicht sensitiv sind, kann man auf die Co-Kultivierung von Agrobacterium mit dem entsprechenden Protoplasten zurückgreifen.

Ti-Plasmide besitzen zwei Regionen, die für die Herstellung transformierter Zellen essentiell sind. Eine davon, die Transfer-DNA Region, wird auf die Pflanze übertragen und führt zur Induktion von Tumoren. Die andere, die virulenzverleihende (vir) Region, ist nur für die Ausbildung, nicht aber für die Aufrechterhaltung der Tumoren essentiell. Die Transfer-DNA Region kann in ihren Dimensionen durch Einbau der Schutzfaktor-Gensequenz vergrössert werden, ohne dass die Uebertragungsfähigkeit beeinträchtigt wird. Durch Entfernen der tumorverursachenden Gene, wodurch die transgenen Pflanzenzellen nicht-tumorös bleiben und durch Einbau eines selektionierbaren Markers, kann das modifizierte Ti-Plasmid als Vektor für den Transfer der erfindungsgemässen Genkonstruktion in eine geeignete Pflanzenzelle verwendet werden.

Die vir-Region bewirkt den Transfer der T-DNA Region von Agrobacterium auf das Genom der Pflanzenzelle, unabhängig davon, ob die T-DNA-Region und die vir-Region auf demselben Vektor oder auf verschiedenen Vektoren innerhalb derselben Agrobacterium-Zelle vorliegen. Eine vir-Region auf einem Chromosom induziert ebenso den Transfer der T-DNA von einem Vektor in eine Pflanzenzelle.

Bevorzugt wird ein System zur Uebertragung einer T-DNA-Region von einem Agrobacterium in Pflanzenzellen, das dadurch gekennzeichnet ist, dass die vir-Region und die T-DNA-Region auf verschiedenen Vektoren liegen. Ein solches System ist unter dem Begriff "binäres Vektor-System" bekannt und der die T-DNA enthaltende Vektor wird als ein "binärer Vektor" bezeichnet.

Jeder T-DNA enthaltende Vektor, der in Pflanzenzellen übertragbar ist und der eine Selektion der transformierten Zellen erlaubt, ist für die Verwendung im Rahmen dieser Erfindung geeignet.

Jeder Vektor mit einer vir-Region, der den Transfer einer T-DNA-Region von Agrobacterium auf Pflanzenzellen bewirkt, kann im Rahmen dieser Erfindung verwendet werden.

Eine weitere Ausführungsform im Rahmen der vorliegenden Erfindung betrifft ein Verfahren, welches die Vorteile einer Virus-vermittelten Transformation und der Transformation mit Hilfe von Agrobacterium verbindet. Dieses Verfahren ist in der Europäischen Patentanmeldung Nr. 201 904 beschrieben. Dabei wird virale DNA oder Teile viraler DNA, die gegebenenfalls Cargo-DNA eingebaut enthalten können, in der Weise in ein T-Replikon integriert, dass ein Einbau der viralen DNA im Pflanzengenom erfolgt.

Der Einbau der viralen DNA ins T-Replikon erfolgt zweckmässigerweise im Bereich der Transfer-DNA-Region des T-Replikon, sodass die virale DNA von mindestens einer der T-DNA Grenzsequenzen flankiert wird, die für die Integration der Transfer-DNA ins pflanzliche Genom essentiell sind.

Auch in diesem Fall kann die Uebertragung der T-DNA-Region von Agrobacterium in eine Pflanzenzelle unter Verwendung eines binären Vektor-Systems erfolgen.

Besonders bevorzugt im Rahmen der vorliegenden Erfindung ist eine hybride genetische Konstruktion, welche die komplette CaMV 35S Enhancer/ Promoter-Region, die Leadersequenz, den Leserahmen ORF VII, die kleine intergenomische Region, 16 Kodons des Leserahmens ORF I, 17 Kodons von Linker- und Spacersequenzen, die für ein Schutzfaktorgen kodierende Region, Spacersequenzen, eine CaMV Polyadenylierungsstelle sowie Sequenzen des Plasmids pUC18 (Norrander J et al, Gene 26: 101 - 106, 1983) enthält.

Besagte hybride gentische Konstruktion kommt zustande durch Verknüpfen eines Eco RV/Eco RI Fragements des CaMV-Stammes CM4.184 (Dixon L et al, Virology, 150: 463 - 468, 1986) welches die 35 S-Promoter-Region, die Leadersequenz, den Leserahmen ORF VII, die kleine intergenomische Region sowie die ersten 16 Kodons des Leserahmens ORF I umfasst, mit dem grossen Sma I/Eco RV Fragment des Plasmids pDH51 (Pietrzak M et al, Nucl. Acids Res., 14: 5857 - 5868, 1986).

Ein für ein Schutzfaktorgen kodierendes DNA-Fragment kann dann mit Hilfe an sich bekannter Methoden in die Polylinker-Region des resultierenden Plasmids eingespleisst werden unter Bildung der oben näher charakterisierten hybriden genetischen Konstruktion.

Eine weitere im Rahmen dieser Erfindung bevorzugte genetische Konstruktion verwendet ein DNA Fragment, welches den 19 S Promotor des Cauliflower Mosaik Virus enthält.
Dieses DNA-Fragment wird zusammen mit dem CaMV Transkriptionsterminator kloniert. In das resultierende Plasmid wird zwischen diese regulatorischen CaMV-Sequenzen der Leseraster eines Strukturgens, vorzugsweise eines für einen Schutzfaktor kodierenden Strukturgens, auf eine Weise eingefügt, dass die Translation besagten Strukturgens direkt am Startkodon des Gen VI Proteins beginnt.

Um dies zu erreichen führt man vorteilhafterweise eine Deletionsmutagenese mit Hilfe eines Oligonukleotids durch.
Dabei geht man von Einzelstrang DNA aus die den entsprechenden Teil des CaMV Genoms umfasst und die mit Hilfe von M13 Helfer-Phagen hergestellt werden kann.
Die resultierende zirkuläre Einstrang-DNA kann dann als Substrat für die in vitro Deletion unter Verwendung von Oligonucleotiden verwendet werden.

In analoger Weise zu der zuvor beschriebenen Konstruktion von in Pflanzen aktiven. Expressionsvektoren unter Verwendung der CaMV-Kontrollsysteme, kann das für Strang-positive (+) RNA-Viren charakteristische und zuvor am Beispiel des BMV-Virus beschriebene Kontrollsystem für die Vektorkonstruktion verwendet werden.

Wie bereits zuvor erwähnt erfolgt bei Strang-positiven (+) RNA-Viren die Herstellung subgenomischer mRNA ausgehend von minus(-)Strang RNA. Im BMV-System wird die Produktion einer subgenomischen RNA 4, die für ein Hüllprotein kodiert, vermutlich durch eine partielle Transkription der minus(-)Strang RNA 3 initiert, unter Beteiligung viruseigener Replikasen.

Durch die Herstellung und Klonierung komplementärer cDNA, ist es möglich, die zuvor im einzelnen besprochenen rekombinanten DNA-Techniken auch für eine genetische Manipulation von RNA-Viren einzusetzen.

In analoger Weise zu dem zuvor besprochenen CaMV-System kann daher auch im Falle Strang-positiver (+) RNA-Viren ein Austausch der für ein virales Protein kodierenden Abschnitte (im Falle von BMV der für das Hüllprotein kodierende Abschnitt auf der RNA 3) mit einem Schutzfaktorgen vorgenommen werden.
Man kann dann diese hybride Genkonstruktion so zwischen in pflanzlichen Zellen aktiven Expressionssignalen einspleissen, dass neben dem Schutzfaktorgen auch die das ursprünglich vorhandene virale Gen kontrollierenden Abschnitte in funktionsfähiger Form erhalten bleiben.
Beim BMV sind dies die stromaufwärts des ORF des ursprünglich vorhandenen Hüllproteingens gelegenen DNA-Abschnitte (French et al., Science, 231: 1294-1297, 1986)

Pflanzenzellen oder Pflanzen, die gemäss der vorliegenden Erfindung transformiert worden sind, können mit Hilfe eines geeigneten phänotypischen Markers, der neben dem Schutzfaktor-Gen und den virusspezifischen Kontrollsequenzen Bestandteil der DNA ist, selektioniert werden. Beispiele solcher phänotypischer Marker, die aber nicht als limitierend aufzufassen sind, beinhalten Antibiotikaresistenz-Marker, wie beispielsweise Kanamycinresistenz- und Hygromycinresistenz-Gene, oder Herbizidresistenz-Marker. Andere phänotypische Marker sind dem Fachmann bekannt und können ebenfalls im Rahmen dieser Erfindung verwendet werden.

Alle Pflanzen, deren Zellen sich durch direkte Einschleusung von DNA durch Verwendung viraler oder bakterieller Vektoren, wie z.B. CaMV oder Agrobacterium oder anderer geeigneter Vektoren transformieren lassen und anschliessend zu vollständigen Pflanzen regenerierbar sind, können dem erfindungsgemässen Verfahren zur Herstellung transgener ganzer Pflanzen, die das übertragene Schutzfaktor-Gen enthalten, unterzogen werden. Es existiert eine ständig wachsende Anzahl von Hinweisen, die darauf schliessen lassen, dass sich im Prinzip alle Pflanzen aus kultivierten Zellen oder Geweben regenieren lassen, einschliesslich, aber nicht beschränkt auf, alle wichtige Getreidearten, Zuckerrohr, Zuckerrüben, Baumwolle, Obstbäumen und anderer Baumarten, Leguminosen sowie Gemüsen.

Das erfindungsgemässe Verfahren eignet sich für die Transformation aller Pflanzen, insbesondere solchen der systematischen Gruppen Angiospermae und Gymnospermae.

Unter den Gymnospermae sind von besonderem Interesse die Pflanzen der Klasse Coniferae.

Unter den Angiospermae sind neben den Laubbäumen und Sträuchern von besonderem Interesse Pflanzen der Familien Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae oder Gramineae und der Ordnung Leguminosae und hier vor allem der Familie Papilionaceae. Bevorzugt sind Vertreter der Solanaceae, Cruciferae, Leguminosae und Gramineae.

Zu den Zielkulturen im Rahmen der vorliegenden Erfindung zählen beispielsweise auch jene, ausgewählt aus der Reihe: Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Gossypium, Lolium, Zea, Triticum und Sorghum, einschliesslich derjenigen, ausgewählt aus der Reihe: Ipomoea, Passiflora, Cyclamen, Malus, Prunus, Rosa, Rubus, Populus, Santalum, Allium, Lilium, Narcissus, Ananas, Arachis, Phaseolus und Pisum.

Diese beispielhafte Aufzählung dient lediglich der näheren Illustrierung der vorliegenden Erfindung und darf nicht als Limitierung des Erfindungsgegenstandes aufgefasst werden.

Durch Anwendung neu entwickelter Transformationstechniken lassen sich mittlerweile in vitro auch solche Pflanzenspezies transformieren, die keine natürlichen Wirtspflanzen für Agrobacterium sind. So sind beispielsweise monokotyle Pflanzen, insbesondere die Getreidearten und Gräser, keine natürlichen Wirte für Agrobacterium.

Es gibt in der Zwischenzeit vermehrt Hinweise darauf, dass sich auch Monokotyledonen mit Agrobacterium transformieren lassen, so dass unter Verwendung von neuen experimentellen Ansätzen, die jetzt verfügbar werden, auch Getreide und Gräser-Spezies einer Transformation zugänglich sind [Grimsley N, et al., Nature, 325: 177-179 (1987)].

Die Regeneration von in Kultur gehaltenen Protoplasten zu ganzen Pflanzen ist bei Evans, et al., "Protoplast Isolation and Culture", in Handbook of Plant Cell Culture, 1: 124-176 (MacMillan Publishing Co. New York 1983); MR Davey, "Recent Developments in the Culture and Regeneration of Plant Protoplasts", Protoplasts, 1983 - Lecture Proceedings, Seite 19-29, (Birkhäuser, Basel 1983); PJ Dale, "Protoplast Culture and Plant Regeneration of Cereals and Other Recalcitrant Crops", in Protoplasts 1983 - Lecture Proceedings, Seite 31-41, (Birkhäuser, Basel 1983); und H Binding, "Regeneration of Plants", in Plant Protoplasts, Seite 21-37, (CRC Press, Boca Raton 1985) beschrieben.

Die Regeneration unterscheidet sich von Pflanzenspezies zu Pflanzen-spezies. Im allgemeinen aber wird zunächst eine Suspension von transformierten Protoplasten, Zellen oder von Gewebe, die zahlreiche Kopien der Schutzfaktor-Gene enthalten, hergestellt. Ausgehend von diesen Suspensionen kann anschliessend die Induktion der Embryobildung erfolgen. Man lässt die Entwicklung der Embryonen bis zum Stadium der Reife und Keimung fortschreiten, wie dies auch bei natürlicherweise vorkommenden Embryonen der Fall ist. Die Kulturmedien enthalten in der Regel verschiedene Aminosäuren und Hormone, wie z.B. Auxin und Cytokinine. Es erweist sich ebenfalls als vorteilhaft, dem Medium Glutaminsäure und Prolin zuzugeben, insbesondere bei Spezies wie Mais und Alfalfa. Die Spross- und Wurzelbildung erfolgt im allgemeinen simultan. Eine effiziente Regeneration hängt in erster Linie vom Medium, vom Genotyp und von der Vorgeschichte der Kultur ab. Werden diese drei Variablen hinreichend kontrolliert, dann ist die Regeneration vollständig reproduzier- und wiederholbar.

Aufgrund neuer Enwicklungen auf dem Gebiet der in vitro Kultivierung von Pflanzen, vornehmlich im Bereich der Pflanzenregeneration, ist es inzwischen möglich, auch bei Vertretern aus der Familie der Gramineae, ausgehend von pflanzlichen Protoplasten, ganze Pflanzen zu regenerieren. Beispiele von erfolgreich durchgeführten Regenerationsexperimenten bei Gramineen sind u.a. bei Abdullah, R et al., Bio/Technology, 4: 1087-1090, 1986, Fujimura, T., et al., Plant Tissue Culture Lett, 2:74-75, 1985, Toriyama, K., et al., Theor Appl Genet, 73:16-19, 1986, Yamada, Y., et al., Plant Cell Rep, 5:85-88, 1986 (Reis) sowie bei Rhodes et al. für Maisprotoplasten (Biotechnology, 6: 56-69, 1988) beschrieben.

Besonders bevozugt im Rahmen dieser Erfindung sind daher auch Vertreter aus der Familie der Gramineae, wie z.B. Pflanzen, die grossflächig angebaut werden und hohe Ernteerträge liefern. Als Beispiele seien genannt: Mais, Reis, Weizen, Gerste, Roggen, Hafer, Hirse oder Sorghum sowie Weidegräser.

Weitere Zielkulturen, die besonders bevorzugt sind für die Verwendung in dem erfindungsgemässen Verfahren, sind daher beispielsweise Pflanzen der Gattungen Allium, Avena, Hordeum, Oryza, Panicum, Saccharum, Secale, Setaria, Sorghum, Triticum, Zea, Musa, Cocos, Phoenix und Elaeis.

Die reifen Pflanzen, die aus transformierten Pflanzenzellen herangezogen wurden, werden zur Samenproduktion mit sich selbst gekreuzt. Einige der Samen enthalten die Gene, die für eine gesteigerte Schutzwirkung verantwortlich sind in einem Verhältnis, das genau den etablierten Gesetzen der Vererbung gehorcht. Diese Samen können zur Produktion virusresistenter Pflanzen verwendet werden. Der Grad der erreichten Virusresistenz der transformierten Pflanzen lässt sich durch künstliche Infektion mit pathogenen Virusstämmen bestimmen.

Homozygote Linien können durch wiederholte Selbstfertilisation und Herstellung von Inzuchtlinien gewonnen werden. Diese Inzuchtlinien können dann wiederum zur Entwicklung von virusresistenten Hybriden verwendet werden. Bei diesem Verfahren wird eine virusresistente Inzuchtlinie mit einer anderen Inzuchtlinie zur Produktion virusresistenter Hybride gekreuzt.

Teile, die von regenerierten Pflanzen erhalten werden können, wie z.B. Blüten, Samen, Blätter, Zweige, Früchte u.a. sind ebenfalls Bestandteil der vorliegenden Erfindung, sofern diese Teile virusresistente Zellen beinhalten. Nachkommen (einschliesslich hybrider Nachkommen), Varietäten und Mutanten der regenerierten Pflanzen sind ebenfalls Bestandteil dieser Erfindung.

Zur Illustration der eher allgemeinen Beschreibung sowie zum besseren Verständnis der vorliegenden Erfindung soll nunmehr auf spezifische Ausführungsbeispiele Bezug genommen werden, die keinen limitierenden Charakter haben, es sei denn, es wird speziell darauf hingewiesen.

### Nichtlimitierende Ausführungsbeispiele:

### Allgemeine rekombinante DNA-Techniken

Da viele der in dieser Erfindung genutzten rekombinanten DNA-Techniken Routine für den Fachmann sind, ist eher hier eine kurze Beschreibung dieser allgemein gebrauchten Techniken enthalten als jedesmal dort, wo sie erscheinen. Bis darauf, wo extra darauf hingewiesen wird, sind alle diese Verfahren in der Referenz von Maniatis et al. (1982) beschrieben.

### A. Schneiden mit Restriktionsendonukleasen

Typischerweise sind etwa 50 bis 500 µg/ml DNA in dem Reaktionsansatz enthalten, in der vom Hersteller, New England Biolabs, Beverly, MA., empfohlenen Pufferlösung. 2 bis 5 Einheiten von Restriktionsendonukleasen werden für jedes µg DNA hinzugefügt und der Reaktionsansatz bei der vom Hersteller empfohlenen Temperatur für eine bis drei Stunden inkubiert. Die Reaktion wird durch 10 minütiges Erhitzen auf 65°C oder durch Extraktion mit Phenol, gefolgt durch Präzipitation der DNA mit Ethanol, beendet. Diese Technik wird auch auf den Seiten 104 bis 106 der Maniatis et al.-Referenz beschrieben.

### B. Behandlung der DNA mit Polymerase, um glatte Enden zu erzeugen

50 bis 500 µg/ml DNA-Fragmente werden zu einem Reaktionsansatz hinzugefügt, in dem vom Hersteller, New England Biolabs, empfohlenen Puffer. Der Reaktionsansatz enthält alle vier Desoxynukleotidtriphosphate in Konzentrationen von 0.2 mM. Die Reaktion erfolgt während 30 Minuten bei 15°C und wird dann durch 10 minütiges Erhitzen auf 65°C beendet. Für Fragmente, die durch Schneiden mit Restriktionsendonukleasen erhalten werden, welche 5'-hervortretende Enden erzeugen, wie EcoRI und BamHI, wird das grosse Fragment, oder Klenow-Fragment, der DNA-Polymerase benutzt. Für Fragmente, die durch Endonukleasen erhalten werden, welche 3'-hervortretende Enden erzeugen, wie PstI und SacI, wird die T4-DNA-Polymerase benutzt. Die Verwendung dieser zwei Enzyme wird auf den Seiten 113 bis 121 der Maniatis et al.-Referenz beschrieben.

### C. Agarose-Gelelektrophorese und Reinigung von DNA-Fragmenten von Gelen

Die Agarose-Gelelektrophorese wird in einem horizontalen Apparat durchgeführt, wie auf den Seiten 150 bis 163 der Maniatis et al.-Referenz beschrieben. Der benutzte Puffer ist der dort beschriebene Tris-Boratpuffer. Die DNA-Fragmente werden durch 0.5 µg/ml Ethidiumbromid gefärbt, das entweder im Gel- oder Tankpuffer während der Elektrophorese vorhanden ist oder nach der Elektrophorese hinzugefügt wird. Die DNA wird durch Beleuchtung mit langwelligem Ultraviolettlicht sichtbar gemacht. Wenn die Fragmente nicht vom Gel abgetrennt werden sollen, wird eine Agarose verwendet, die bei niedriger Temperatur geliert und von Sigma Chemical, St. Louis, Missouri, bezogen werden kann. Nach der Elektrophorese wird das gewünschte Fragment ausgeschnitten, in ein Plastikröhrchen gegeben, etwa 15 Minuten auf 65°C erhitzt, dreimal mit Phenol extrahiert und zweimal mit Ethanol gefällt. Dieses Verfahren ist gegenüber dem von Maniatis et al. auf Seite 170 beschriebenen leicht verändert. Als Alternative kann die DNA aus der Agarose mit Hilfe des Geneclean Kits (Bio 101 Inc., La Jolla, CA, USA) isoliert werden.

### D. Hinzufügen von synthetischen Linkerfragmenten an DNA-Enden

Wenn es erwünscht ist, eine neue Endonukleaseschnittstelle an das Ende eines DNA-Moleküls anzufügen, wird das Molekül gegebenenfalls zuerst mit DNA-Polymerase behandelt, um glatte Enden zu erzeugen, wie im obigen Abschnitt beschrieben. Etwa 0.1 bis 1.0 µg dieses Fragments wird zu etwa 10 ng phosphorylierter Linker-DNA gegeben, die von New England Biolabs bezogen wurde, in einem Volumen von 20 bis 30 µl mit 2 µl T4 DNA-Ligase von New England Biolabs, und 1mM ATP in dem vom Hersteller empfohlenen Puffer. Nach einer Inkubation über Nacht bei 15°C wird die Reaktion durch 10 minütiges Erhitzen auf 65°C beendet. Der Reaktionsansatz wird auf etwa 100 µl in einem Puffer verdünnt, der richtig für die Restriktionsendonuklease ist, die die synthetische Linkersequenz schneidet. Ungefähr 50 bis 200 Einheiten dieser Endonuklease werden hinzugefügt. Die Mischung wird 2 bis 6 Stunden bei der angemessenen Temperatur inkubiert, dann wird das Fragment einer Agarose-Gelelektrophorese unterworfen und gereinigt wie oben beschrieben. Das resultierende Fragment wird nun Enden haben mit Endigungen, welche durch Schneiden mit der Restriktionsendonuklease erzeugt wurden. Diese Enden sind gewöhnlich kohäsiv, so dass das resultierende Fragment nun leicht an andere Fragmente mit den gleichen kohäsiven Enden geknüpft werden kann.

### E. Entfernen von 5'-terminalen Phosphaten von DNA-Fragmenten

Während der Plasmidklonierungsschritte vermindert die Behandlung des Vektorplasmids mit Phosphatase die Rezirkularisation des Vektors (diskutiert auf Seite 13 der Maniatis et al.-Referenz). Nach Schneiden der DNA mit der richtigen Restriktionsendonuklease wird eine Einheit alkalische Phosphatase aus dem Darm von Kälbern hinzugefügt, die von Boehringer-Mannheim, Mannheim, erworben wurde. Die DNA wird eine Stunde bei 37°C inkubiert und anschliessend zweimal mit Phenol extrahiert und mit Ethanol gefällt.

### F. Verknüpfen der DNA-Fragmente

Wenn Fragmente mit komplementären kohäsiven Enden miteinander verknüpft werden sollen, werden etwa 100 ng von jedem Fragment in einem Reaktionsgemisch von 20 bis 40 µl mit etwa 0.2 Einheiten T4 DNA-Ligase von New England Biolabs im vom Hersteller empfohlenen Puffer inkubiert. Die Inkubation wird 1 bis 20 Stunden lang bei 15°C durchgeführt. Wenn DNA-Fragmente mit glatten Enden verknüpft werden sollen, werden sie inkubiert wie oben, bis darauf, dass die Menge der T4 DNA-Ligase auf 2 bis 4 Einheiten erhöht wird.

### G. Die Transformation von DNA in E. coli

E. coli Stamm HB101 wird für die meisten Experimente verwendet. DNA wird mit dem Kalziumchloridverfahren, wie es von Maniatis et al., Seiten 250 bis 251, beschrieben wurde, in E. coli eingeführt.

### H. Screening von E. coli auf Plasmide

Nach der Transformation werden die resultierenden Kolonien von E. coli auf das Vorhandensein des gewünschten Plasmids durch ein schnelles Plasmidisolationsverfahren geprüft. Zwei gebräuchliche Verfahren werden auf den Seiten 366 bis 369 der Maniatis et al.-Referenz beschrieben.

### I. Isolierung von Plasmid-DNA in grossem Massstab

Verfahren zur Isolierung von Plasmiden aus E. coli in grossem Massstab werden auf den Seiten 88 bis 94 der Maniatis et al.-Referenz beschrieben.

### J. Klonierung in M13 Phagenvektoren

Die folgende Beschreibung ist so zu verstehen, dass die doppelsträngige replikative Form der Phage M13-Abkömmlinge für Routineverfahren, wie Schneiden mit Restriktionsendonuklease, Verknüpfen etc., benutzt wird.

### Beispiel 1: Transaktivierung eines CAT-Gens in Tabak-Protoplasten

### 1.1. Konstruktion des Plasmids pHS1

Das EcoRV/EcoRI Fragment des CaMV Stammes CM4.184 (Dixon L et al, Virology, 150: 463 - 468, 1986), welches die 35S-Promotor-Region, die Leadersequenz, den Leserahmen ORF VII, die kleine intergenomische Region sowie die ersten 16 Kodons des Leserahmens ORF I umfasst, wird aus einem Polyacrylamid-Gel heraus isoliert und anschliessend zur Ergänzung der EcoRI kohesiven Enden mit dem Klenow-Fragment der DNA-Polymerase (abgekürzt "Klenow") behandelt.

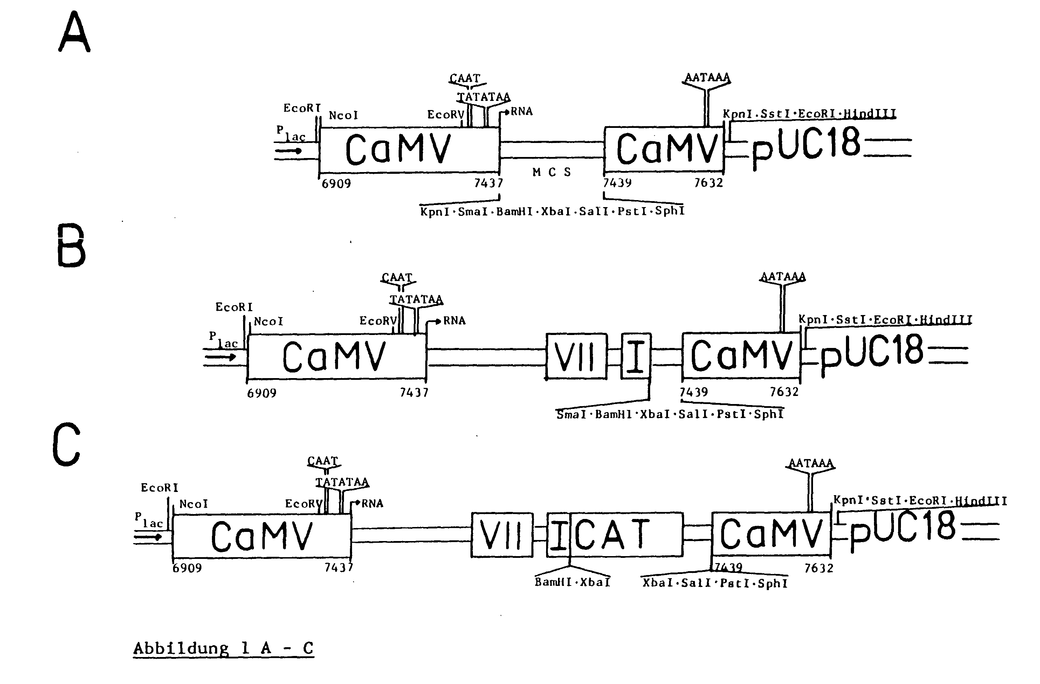

Dieses EcoRV/EcoRI Fragment wird mit dem grossen SmaI/EcoRV Fragment des Plasmids pDH51, das bei Pietrzak et al, 1986 (Pietrzak M et al, Nucl. Acids Res, 14: 5857 - 5868, 1986) beschrieben ist, verknüpft (Abb. 1A). Dieses Konstrukt wird in dem E. coli Stamm DH1 kloniert. Anschliessend werden mit Hilfe der Restriktionsanalyse diejenigen Transformanten ermittelt, bei denen die EcoRV Restriktionsstelle wiederhergestellt ist, während die SmaI-Stelle fehlt und welche Restriktionsfragmente mit einer entsprechenden Grösse aufweisen (Abb. 1B).

Das resultierende Plasmid wird dann im Bereich der Polylinker-Region mit XbaI geschnitten und die kohesiven Enden werden mit Hilfe der "Klenow" ergänzt. Ein Hind III-Fragment, welches die kodierende Region der bakteriellen Chloramphenicoltransacetylase (CAT) trägt (Alton NK et Vapnek D, Nature, 282: 864 - 869, 1979) wird isoliert und die kohesiven Enden werden mit "Klenow" aufgefüllt.

Die ergänzten Hind III und XbaI-Fragmente werden anschliessend miteinander verknüpft und für die Transformation des E. coli Stammes DH1 verwendet. Die resultierenden Transformanten, die eine wiederhergestellte XbaI-Restriktionsstelle besitzen sowie die richtige Orientierung des Insert aufweisen, werden ausgewählt. Die geeignete genetische Konstruktion besitzt demnach die komplette CaMV 35S Enhancer/Promoter-Region, die Leadersequenz, den Leserahmen ORF VII, die kleine intergenomische Region, 16 Kodons des Leserahmens ORF I, 17 Kodons von Linker-und Spacersequenzen, die kodierende Region des bakteriellen CAT Gens, Spacersequenzen, eine CaMV Polyadenylierungsstelle sowie Sequenzen des Plasmids pUC18 (Norrander J et al, Gene 26: 101 - 106, 1983).

Die ORF I, Spacer- und CAT-Kodons bilden zusammen einen fusionierten Leserahmen. Das resultierende Plasmid verleiht transformierten Pflanzenprotoplasten eine CAT-Aktivität, die bei 10 % - 20 % liegt im Vergleich zum Kontrollplasmid pDW2 (Pietrzak et al, 1986, supra).

### 1.2. Transformation von Tabak-Protoplasten

Tabakprotoplasten von Nicotiana tabacum c.v. Petite Havana werden nach herkömmlichen Verfahren ausgehend von einer Tabak-Suspensionskultur hergestellt (Potrykus I und Shillito RD, Methods in Enzymology, Vol 118, Plant Molecular Biology, eds. A. und H., Weissbach, Academic Press, Orlando, 1986) vollständig entfaltete Blätter von 6 Wochen alten Spross-Kulturen werden unter sterilen Bedingungen entfernt und mit einer Enzym-Lösung der folgenden Zusammensetzung gründlich benetzt.

| | | |
|---|---|---|
| Enzymlösung: | H₂O | 70 ml |
| | Sucrose | 13 g |
| | Macerozyme R 10 | 1 g |
| | Cellulase | 2 g |
| | 'Onozuka' R 10 (Yakult Co. Ltd., Japan) | |
| | Drisellase (Chemische Fabrik Schweizerhalle, Schweiz) | 0.13 g |
| | 2(n-Morpholin)-ethansulfonsäure (MES) | 0.5 ml |
| | pH 6.0 | |

Man zerschneidet Blätter anschliessend in 1 - 2 cm grosse Quadrate und lässt sie auf der oben genannten Enzymlösung aufschwimmen. Die Inkubation erfolgt über Nacht bei einer Temperatur von 26°C im Dunkeln. Dieser Ansatz wird anschliessend leicht gerührt und für weitere 30 min bis zur vollständigen Verdauung inkubiert.

Diese Suspension wird dann durch ein Stahlsieb mit einer Maschenweite von 100 um filtriert, mit 0.6 M Sucrose (MES, pH 5.6) durchgespült und anschliessend 10 Minuten bei 4000 - 5000 rpm zentrifugiert. Die Protoplasten sammeln sich auf der Oberfläche des Mediums, das dann unter den Protoplasten abgezogen wird, z.B. unter Verwendung einer sterilisierten Injektionsspritze.

Die Protoplasten werden in einem K3A Medium [Sucrose (102,96 g/l; Xylose (0.25 g/l); 2.4 D (0.10 mg/l); NAA (1.00 mg/l); BAP (0.20 mg/l); PH 5.8] resuspendiert, das 0.4 M Sucrose enthält.

Die Protoplasten werden anschliessend durch Flotation der Protoplasten und Entfernen des Mediums in der zuvor beschriebenen Weise gewaschen (3x).

Die Transformation der Protoplasten mit Plasmid-DNA erfolgt mit Hilfe des direkten Gentransfers (Paszkowski J et al, EMBO J., 3: 2717 - 2722, 1984) unter Verwendung der Elektroporationstechnik (Fromm M et al, Proc. Natl. Acad. Sci, USA, 82: 5824 - 5829, 1985).

Die Elektroporation der Tabakprotoplasten wird in einer mit Aluminiumfolie ausgelegten Wegwerf-Halbmicrokuvette (Greiner, Nürnberg) durchgeführt.

In besagter Küvette werden die Tabakprotoplasten in einer Populationsdichte von 2 x 10⁶ Protoplasten/ml in 0.7 ml Elektroporationspuffer (Fromm et al, 1985, supra) der folgenden Zusammensetzng:
Endkonzentration
10 mM Hepes pH 7.2
-150 mM NaCl
-5 mM CaCl₂
-0.2 M mannitol
zusammen mit 15 µg Plasmid-DNA unter sterilen Bedingungen inkubiert.

Die Elektroporation der Protoplasten erfolgt durch Entladung einer Kapazität von 820 µF, die zuvor bei 220 V aufgeladen wurde (Fromm et al, 1985, supra).

Nach einer Inkubationszeit von 10 min. bei 4°C und einer anschliessenden Inkubation für weitere 10 min bei Zimmertemperatur, werden die behandelten Protoplasten mit 10 ml AA Medium der folgenden Zusammensetzung verdünnt:
AA-Medium für 1 1
   - 37 g/l MgSO₄ 10 ml
   - 17 g/l KH₂PO₄ 20 ml
   - 44 g/l CaCl₂ 10 ml
   - KCl 2.95 g
   - (¹MS) EDTA 5 ml
   - (MS) FeCl₃ 5 ml

   - (MS or NT) Mikroelemente 1 ml
   - 1000x Vitamine (siehe unten) 1 ml
   - 100x Aminosäuren (siehe unten) 10 ml

   - 100x Mikrozucker (siehe unten) 10 ml
   - Sorbitol 63 g

   - ²2,4-D, 20 mg/100 ml 5 ml
   - Kinetin, 20 mg/100 ml 1 ml
   - ³GA3, 20 mg/100 ml 0.5 ml

   - pH 5.8-6 (NaOH).
      osmotischer Druck 530-570 mOs
1000x Vitamine (100 ml):
   - Inositol 2 g
   - Nicotinsäure 50 mg
   - Thiamin 40 mg
   - Pyridoxin 10 mg
   - MES 1 M pH 7 1 ml
   Auffüllen mit H₂O auf 100 ml
100x Aminosäure Mix:
   - Glutaminsäure 43.8 g
   - Asparagin 13.3 g
   - Arginin-HCl 8.7 g
   - Glycin 0.375 g

   - pH ca. 6,
   Auffüllen mit H₂O auf 500 ml
100x Mikrozucker-Mix:
   - Xylose 1.5 g
   - Arabinose 1.5 g
   - Glukose 1.8 g
   - Inositol 0.8 g
   Auffüllen mit H₂O auf 100 ml

¹ MS = Murashige and Shook Medium (Murashige T and Skoog F, Physiol. Plant, 15: 473, 1962)
² 2,4-D 2,4-Dichlorphenoxiessigsäure
³ GA3 Gibberellinsäure

Die verschiedenen Lösungen werden durch Autoklavieren (Aminosäure-Mix) oder durch Filtersterilisation sterilisiert.

Es folgt eine 2-tägige Inkubation der Protoplasten bei 28°C im Dunkeln. Die Zellen werden dann entsprechend der bei Fromm et al, 1985, supra beschriebenen Methode unter Verwendung von Ultraschall lysiert und extrahiert.

### 1.3. CAT-Assay

Der so gewonnene Extrakt wird anschliessend mittels Dünnschichtchromatographie auf seine Fähigkeit zu Acetylierung von radioaktivem Chloramphenicol hin untersucht (CAT-Aktivität, Gormann M et al, Mol. Cell. Biol., 2: 1044 - 1051, 1982).

### 1.4. ERGEBNISSE:

Die Ergebnisse des CAT-Assays machen deutlich, dass in den mit dem Plasmid pDW2 behandelten Protoplasten radioaktiv markiertes Acetylchloramphenicol im Chromatogramm nachweisbar ist, d.h. dass die Protoplasten eine CAT-Aktivität beinhalten.

In mit dem Plasmid pHS1 behandelten Zellen liess sich dagegen keine entsprechende Aktivität nachweisen.

Wird die Elektroporationsmischung, enthaltend Tabak-Protoplasten und das Plasmid pHS1, zusätzlich mit 15 µg CaMV DNA versetzt, dann zeigen auch die pHS1 behandelten Zellen CAT Aktivität.

Auf gleiche Weise durchgeführte Kontrollexperimente unter Verwendung von Kalbsthymus DNA erbrachten dagegen negative Resultate (keine CAT-Aktivität).

Diese Ergebnisse zeigen damit eindeutig, dass eine Expression in der Position des CAT-Leserasters im Plasmid pHS1 nur in Anwesenheit von Virus-DNA erfolgt und dass somit eine Induktion der CAT-Aktivität durch Verwendung von Virus-DNA möglich ist.

Anstelle des CAT-Gens im Plasmid pHS1 kann jedes beliebige im Rahmen der vorliegenden Erfindung geeignete Gen, wie beispielsweise ein Schutzfaktorgen, eingesetzt werden.

### Beispiel 2: Transaktivierung eines CAT-Gens in Brassica napus-Pflanzen

### 2.1. Konstruktion des Plasmids pCIB 200

TJS75kan wird durch Verdauen des Plasmids pTJS75 (Schmidhauser und Helinski, J. Bacteriol., 164: 446-455, 1985) mit dem Restriktionsenzym NarI zur Gewinnung des Tetracyclinresistenzgens und anschliessende Insertion eines AccI Fragmentes von pUC4K (Vierra und Messing, Gene, 19: 259-268, 1982), das das NptI-Gen trägt, hergestellt. pCIB200 entsteht dann durch Verknüpfen von XhoI-Linkern mit dem EcoRV-Fragment von pCIB7 [dieses Fragment enthält die linke und rechte T-DNA Grenzsequenz, ein chimäres nos/nptII Gen sowie die pUC Polylinkerregion (Rothstein SJ et al., Gene, 53: 153-161, 1987)] und Klonierung des XhoI verdauten Fragmentes in die SalI Schnittstelle von TJS75kan.

### 2.2. Herstellung des kointegrierten Vektors pCIB200/HS1

Das Plasmid pCIB200, dessen Konstruktion in Beispiel 2.1 beschrieben ist und Plasmid pHS1 aus Beispiel 1.1 werden als Ausgangsmaterial für die Herstellung des kointegrierten Vektors pCIB200/HS1 verwendet.

Beide Elternplasmide werden zunächst mit dem Restriktionsenzym KpnI geschnitten, miteinander in einem der üblicherweise verwendeten Inkubationspuffer gemischt und anschliessend unter Zugabe geeigneter Enzyme (Ligasen) miteinander verknüpft.

Anschliessend werden E. coli HB101-Zellen mit diesem Gemisch nach der bei Maniatis et al., 1982 beschriebenen Methode (vgl. Maniatis et al., Seiten 250-251) transformiert und Ampicillin/Kanamycin resistent E. coli Kolonien selektioniert.

Resistente Kolonien werden mit Hilfe einer Restriktionsanalyse auf Anwesenheit des gewünschten kointegrierten Plasmids pCIB200/HS1 hin überprüft, indem man dieses durch KpnI wieder in die linearen Formen der Elternplasmide zerlegt.

### 2.3. Ueberführen des kointegrierten Vektors pCIB200/HS1 in Agrobacterium tumefaciens durch "triparental mating"

Als Ausgangsmaterial (Eltern) für das "triparental mating" verwendet man E. coli HB101 (pRK2013) [beschrieben bei Tepfer und Casse-Delbart, Microbiol. Sci., 4: 24, 1987], E. coli HB101 (pCIB200/HS1), das den oben beschriebenen Kointegrationsvektor pCIB200/HS1 enthält sowie Agrobacterium tumefaciens C58 (pCIB542).

Bei dem Plasmid pCIB542 handelt es sich um ein apathogenes Helferplasmid, dessen Konstruktion in der Europäischen Patentanmeldung EP 256 223 ausführlich beschrieben ist und die in Form einer Referenz ein Bestandteil vorliegender Erfindung ist.

Die Kreuzung der 3 Eltern-Stämme und damit die Ueberführung des kointegrierten Plasmids pCIB200/HS1 in Agrobacterium erfolgt nach dem bei Tepfer und Casse-Delbart, 1987 beschriebenen Verfahren.
Die aus diesem "triparental mating" resultierenden Agrabakterien erhalten die Bezeichnung C58 (pCIB542: pCIB200/HS1). Sie besitzen damit neben dem Helferplasmid pCIB542 auch das kointegrierte Plasmid pCIB200/HS1.

### 2.4. Herstellung transgener Brassica napus Pflanzen

Blattstengelstückchen (Petiolen) von Brassica napus werden nach dem bei Guerche et al., Mol. Gen. Genet., 206: 382-386 1987 beschriebenen Verfahren transformiert. Anstelle der dort verwendeten Agrobacterium rhizogenes-Stämme erfolgt die Transformation von Brassica napus in diesem Fall mit Hilfe eines Gemisches von Agrobacterium rhizogenes und Agrobacterium tumefaciens C58 (pCIB542: pCIB200/HS1), dessen Herstellung in Beispiel 2.3. beschrieben ist. Das Mischungsverhältnis von A. rhizogenes und A. tumefaciens C58 (pCIB542: pCIB200/HS1) beträgt vorzugsweise 9:1.

### 2.4.1. Kultivierung der Agrobacterium-Stämme und Herstellung der Inokulationslösung

Vor der Inokulation werden die Agrobacterien-Stämme auf YEB Medium [Bacto Rindfleischextrakt 5 g/l, Bacto Hefeextrakt 1 g/l, Peptone 5 g/l, Sucrose 5 g/l MgSO₄ 2 mM, pH 7,2], welches zuvor mit 100 µg/ml Ampicillin und 25 µg/ml Kanamycin angereichert und mit 1,5 % Agar verfestigt wurde, ausplattiert. Nach einer Kultivierungsdauer von 48 h bei einer Temperatur von 28°C wird eine einzelne Kolonie zum Animpfen einer Flüssigkultur verwendet. Diese wird in 100 ml Erlenmeyer-Kolben in einem flüssigen YEB-Medium, welches mit Antibiotika in der zuvor angegebenen Konzentration angereichert ist, durchgeführt. Die Kultivierung erfolgt bei einer Temperatur von 28°C auf einer Schüttelmaschine bei einer Geschwindigkeit von 200 U.p.m. Die Kultivierungsdauer beträgt 24 h.

Anschliessend wird eine zweite Subkultivierung in flüssigem Medium mit einem Verdünnungsverhältnis von 1:20 unter ansonsten gleichen Bedingungen durchgeführt. Die Inkubationsdauer beträgt in diesem Fall 20 h.

Diese Massnahmen führen zu einer Populationsdichte an lebenden Agrobakterien von etwa 10⁹/ml.

Die Bakterienzellen werden durch Abzentrifugieren geerntet und anschliessend in einem äquivalenten Volumen einer 10 mM MgSO₄-Lösung, die keine Antibiotika enthält, resuspendiert.

### 2.4.2. Inokulation von Brassica napus Blattstengelstückchen

Blattstengel von Brassica napus werden zunächst 10 min in einer Kalziumhypochlorit-Lösung (70 g/l) oberflächensterilisiert und mit destilliertem Wasser abgespült. Anschliessend werden diese in kleine Stücke geschnittene, die eine Dicke von 6-10 mm aufweisen und in Petri-Schalen überführt. Diese enthalten steriles Leitungswasser oder aber eine Salzlösung nach Monnier (Monnier, Revue des Cytologie et de Biologie Végetales, tome 31: 78, 1976), die durch Zugabe von 0,7 % Agar verfestigt wird.

Für die Inokulation werden gut gewachsene Agrobacterienkulturen verwendet, die eine OD₆₈₀ von ca. 1.00 aufweisen (dies entspricht einer Anzahl von ca. 10⁹ Zellen/ml).

Für die gemischte Inokulation von Agrobacterium rhizogenes und Agrobacterium tumefaciens werden die Bakterien zunächst bis zum Erreichen der erforderlichen Zelldichte getrennt kultiviert und erst unmittelbar vor der beabsichtigten Inokulation gemischt und zwar in einem Verhältnis von 9:1 (A. rhizogenes:A. tumefaciens).

Nach erfolgter Inokulation werden die Petri-Schalen gut verschlossen und für etwa 2 bis 3 Wochen bei Raumtemperatur inkubiert. Eine erfolgreiche Transformation mit A. rhizogenes lässt sich dann sehr einfach am Auftreten haariger Würzelchen erkennen.

Die Transformation durch A. tumefaciens kann dagegen nur anhand der erworbenen Kanamycin-Resistenz nachgewiesen werden.

Bei dem hier verwendeten Mischungsverhältnis von 9:1 enthalten bis zu 95 % der Würzelchenkolonien eine durch A. tumefaciens-Transformation verursachte Kanamycin-Resistenz.

### 2.5. Regeneration ganzer Pflanzen

Die Regeneration ganzer Pflanzen aus Wurzelkolonien erfolgt nach bekannten Verfahren, wie sie etwa bei David et al., Biotechnology, 1: 73, 1982, Tepfer und Casse-Delbart, 1987 oder bei Guerche et al., Mol. Gen. Genet., 206:382-386 1987 beschrieben sind.

Wurzelgewebe wird auf ein Monnier Medium überführt, das 0,36 µM 2,4 D und 0,72 µM Kinetin als Zusatz enthält und mit 0,8 % Agar verfestigt wird. Nach 4 Wochen Kulturdauer wird das entstandene Kallusgewebe in ein flüssiges, hormonfreies Medium nach Monnier überführt und auf einer Rundschüttelmaschine bei 150 Upm und einer Temperatur von 22°C bis 25°C inkubiert.
Der Kallus löst sich auf und bildet eine Suspensionskultur, aus der sich nach ca. 1 Monat Embryonen differenzieren. Diese Embyronen werden in Petrischalen auf hormonfreies Monnier-Medium weiterkultiviert, wo sie sich in kleine Pflänzchen weiterdifferenzieren.

### 2.6. Transaktivierung transgener Pflanzen durch CaMV-Infektion

Transgene Brassica napus Pflanzen werden nach Lebeurier et al., 1980 mit Cauliflower Mosaik Virus infiziert.

Die CAT-Aktivität vor und nach der erfolgten CaMV-Infektion wird in Blattextrakten transgener Brassica napus Pflanzen nach dem bei Fromm et al., 1982 beschriebenen Verfahren bestimmt.

Dabei wird zunächst Blattmaterial (50 mg bis 100 mg) in einem Extraktionspuffer (10 % v/v Glycerol, 0,01 % w/v Natriumdodecylsulfat, 5 % v/v Mercaptoethanol, 0,005 % Bromphenolblau, 0,0625 M Tris pH 6,8) zerkleinert. Der partikuläre Teil des so gewonnenen Extrakts wird mit Hilfe einer Zentrifugation beseitigt.
Der Ueberstand wird bei einer Temperatur von 65°C für 10 min erhitzt (Inaktivierung pflanzlicher Substanzen, die die CAT-Aktivität beeinträchtigen), anschliessend auf Raumtemperatur abgekühlt und in dem unter Punkt 1.3. beschriebenen CAT-Assay verwendet. Die mit dem HS1-Konstrukt transformierten Pflanzen zeigen eine erhöhte Aktivierbarkeit des CAT-Gens, die bis zum 50fachen im Vergleich zu den Kontrollen betragen kann.

### Beispiel 3: Transaktivierung von CaMV 19 S Transkripten

### 3.1. Konstruktion von p19SCAT

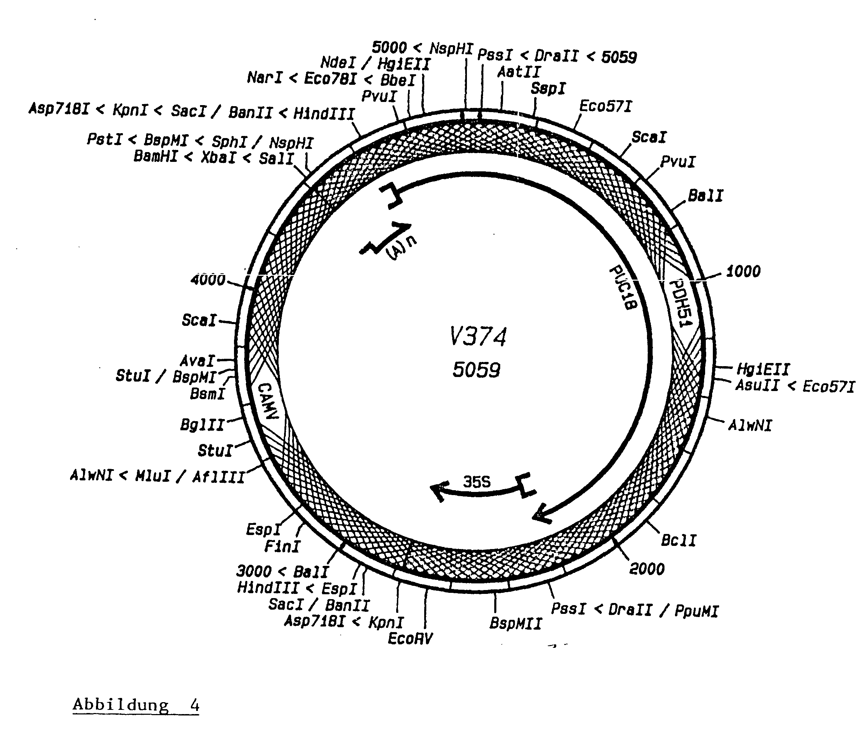

Als Vektor wird das Plasmid pDH19u verwendet, das aus der CaMV 35S Promotor-Terminator Kassette aus dem Plasmid pDH51 (Pietrzak M et al., Nucl. Acids Res., 14: 5857-5868, 1986) und dem Plasmid pTZ19u (Mead, DA, et al., B., Prot. Engineering, 1: 67-74, 1986) zusamengesetzt ist. Dazu wird pDH51 mit EcoRI verdaut, die resultierenden Fragmente mit T4 DNA Polymerase behandelt und das CaMV 35S Promotor-Terminator Fragment in das grosse PvuII Fragment von pTZ19u kloniert.
Nach Verdauung von pDH19u mit NcoI und SmaI wird das überhängende NcoI-Ende mit Hilfe von "Klenow" DNA Polymerase aufgefüllt und das grosse Fragment isoliert. Als CaMV 19S Promoter Fragment wird die Genomregion von Position 4875 bis Position 5355 von CaMV (Stamm CM4.184, Dixon L et al., Virology, 150: 463-468, 1986) als MnlI Fragment isoliert und mit dem grossen NcoI/SmaI Fragment von pDH19u verknüpft. Dadurch·wird das Plasmid p19S erhalten, das den CaMV 19S Promotor, Polylinker-sequenzen und den CaMV Transkriptionsterminator enthält.
Plasmid p19S wird mit BamHI und SalI geöffnet und mit dem kleinen BamHI/SalI CAT-Fragment aus Plasmid pZL811 (Wong, DT et al., J.Virol., 55: 223-231, 1985) verknüpft. Im letzten Schritt wird das ATG Starkodon des CAT Leserasters durch Deletionsmutagenese mit Hilfe eines Oligonukleotides mit dem ATG Startkodon des CaMV Proteins VI fusioniert.
Als Transaktivatorplasmid wurd das Plasmid V374 (siehe Abbildung 4) verwendet. Es enthält das EcoRV Fragment aus CaMV (Stamm CM4.184), das das Gen VI enthält, kloniert in die SmaI Restriktionsstelle von Plasmid pDH51.

### 3.2. Transformation von Brassica rapa Protoplasten

Turgeszente Blätter von Brassica rapa werden zunächst oberflächensterilisiert durch Einlegen in eine 0,5 % Kalziumhypochlorit-Lösung für 30 Minuten Anschliessend werden die Blätter mit sterilem Wasser für jeweils 1 bis 2 Minuten gewaschen (5x).
Die so behandelten Blätter werden in grobe Stücke geschnitten und in eine Enzymlösung eingebracht und dort in ca. 1 mm breite Streifen geschnitten. Die Inkubation erfolgt über Nacht im Dunkeln bei einer Temperatur von 26°C.

| | | |
|---|---|---|
| Enzymlösung: (100 ml) | Mannit | 6,15 g |
| | CaCl₂ | 0,63 g |
| | Cellulose "Onozuka" R10 (Yakult Co., Ltd., Japan) | 1,0 g |
| | Macerozyme R10 | 0,1 g |
| | pH | 5,4 |

Nach der Uebernacht-Inkubation wird die Enzymlösung, enthaltend Protoplasten und Blattreste, auf eine 50 um Stahlsieb gegeben und filtriert. Die so abgetrennten Protoplasten werden in Zentrifugenröhrchen überführt und 10 min bei 4000-5000 rpm zentrifugiert. Der Ueberstand (Enzymlösung) wird verworfen und die pelletierten Protoplasten in 10 ml Elektroporationspuffer wiederaufgenommen. Der ganze Vorgang wird nochmals wiederholt und die Protoplasten-Suspension auf 3 x 10⁶/ml eingestellt.

Die Elektroporation der Brassica rapa Protoplasten erfolgt in analoger Weise zu den in Beispiel 1.2. für Tabak-Protoplasten beschriebenen Verfahren, jedoch erfolgt in diesem Fall die Aufladung der Kondensatoren mit 180 V.

Die Inkubation der elektroporierten Brassica-Protoplasten wird in A Medium (siehe unten) antselle des für Tabak-Protoplasten verwendeten AA Mediums durchgeführt.

| A-Medium 1 1: | |
|---|---|
| A macro | 100 ml |
| A micro ≙ B5 micro | 1 ml |
| MS Eisen | je 5 ml |
| A vita ≙ B5 vita | 10 ml |
| A OS | 25 ml |
| Glucose | 80 g/l |
| Xylose | 250 mg/l |
| Caseinhydrolysate (= N-Z-amine) | 500 mg/l |
| ⁴2,4 D | 1 mg/l |
| ⁵NAA | 0,1 mg/l |
| ⁶6BAP | 0,5 mg/l |
| pH | 5,7-5,8 |

| Stammlösungen (A-Medium): | |
|---|---|
| A macro (per 100 ml): | |
| NaH₂PO₄·1 | 75 mg |
| KH₂PO₄ | 170 mg |
| KNO₃ | 2200 mg |
| NH₄NO₃ | 600 mg |
| (NH₄)₂SO₄ | 75 mg |
| MgSO₄·7 | 310 mg |
| CaCl₂·2 | 295 mg |

| A micro (per 100 ml): | |
|---|---|
| MnSO₄·1 | 1000 mg |
| Na₂MoO₄·2 | 25 mg |
| H₃BO₃ | 300 mg |
| ZnSO₄·7 | 200 mg |
| CuSO₄·5 | 2,5 mg |
| CoCl₂·6 | 2,5 mg |
| KJ | 75 mg |

| A vita (per 100 ml): | |
|---|---|
| Inositol | 1000 mg |
| Nicotinsäure | 10 mg |
| Pyridoxin·HCl | 10 mg |
| Thiamin·HCl | 100 mg |

| A OS (per 100 ml): | |
|---|---|
| Natriumpyruvat | 20 mg |
| Zitronensäure | 40 mg |
| Aepelsäure | 40 mg |
| Fumarsäure | 40 mg |
| (pH auf 5,5 einstellen mit NH₄OH) | |

| | |
|---|---|
| ⁴ 2,4 D 2,4-Dichlorphenoxiessigsäure | |
| ⁵ NAA Naphthyl-1-essigsäure | |
| ⁶ 6BAP 6-Benzylaminopurin | |

### 3.3. Ergebnisse

Die induzierte CAT Aktivität wird 20 Stunden nach der erfolgten Elektroporation gemessen unter Verwendung der in Abschnitt 1.3. beschriebenen CAT-Assays. Die Analyse ergibt, dass die Elektroporation mit p19SCAT nur zu einer sehr niedrigen CAT Expression führt. Wird jedoch zusammen mit p19SCAT ein Plasmid in die Protoplasten elektroporiert, von dem das CaMV Protein VI exprimiert werden kann, dann lässt sich eine etwa 20fache Stimulierung der CAT Aktivität beobachten.

## Patentansprüche

1. Verfahren zum Schutz von Pflanzen vor einer Virusinfektion und deren Folgen auf der Grundlage viruseigener Kontrollmechanismen, **dadurch gekennzeichnet**, dass man
(a) eine DNA Sequenz, welche ein antiviral oder zelltoxisch wirkenden Schutzfaktor kodiert, mit einer cis-aktiven virusspezifischen Kontrolregion, die über einen auf der Transkriptions- oder Posttranskriptions bzw. -translationsebene wirksamen Transaktivierungsmechanismus reguliert wird, sowie gegebenfalls mit in der pflanzlichen Zelle wirksamen Expressionssignalen operabel verknüpft; und
(b) das chimäre Konstrukt gemäss (a) in die zu schützende Pflanze einbringt, sodass es nach Infektion der Pflanze mit viraler DNA zu einer virusinduzierten Expression des Schutzfaktorgens kommt.

2. Verfahren gemäss Anspruch 1, **dadurch gekennzeichnet**, dass die virusspezifische Induktion der Expression der Schutzfaktor-kodierenden DNA Sequenz über eine Transaktivierung der Transkription erfolgt.

3. Verfahren gemäss Anspruch 1, **dadurch gekennzeichnet**, dass die virusspezifische Induktion der Expression der Schutzfaktor-kodierenden DNA Sequenz auf der posttranskriptionalen Ebene erfolgt.

4. Verfahren gemäss Anspruch 3, **dadurch gekennzeichnet**, dass die virusspezifische Induktion der Expression der Schutzfaktor-kodierenden DNA Sequenz über
a) eine Kontrolle der mRNA Verlängerung,
b) eine Kontrolle des mRNA Transports vom Nukleus ins Cytoplasma,
c) eine Kontrolle des mRNA Spleissens,
d) eine Kontrolle der mRNA Polyadenylierung,
e) eine Kontrolle der Translations-Initiation oder
f) eine differenzierte Verwendung verschiedener ORF's auf einer polycistronischen RNA erfolgt.

5. Verfahren gemäss Anspruch 1, **dadurch gekennzeichnet**, dass die virusspezifische Induktion der Expression der Schutzfaktor-kodierenden DNA Sequenz durch posttranslationale Modifikation des Protein-Produktes erfolgt.

6. Verfahren gemäss Anspruch 1, **dadurch gekennzeichnet**, dass man die induzierbare Schutzfaktor-kodierende DNA Sequenz in operabler Weise mit Expressionssignalen verknüpft, die in der pflanzlichen Zelle aktiv sind und die die Expression besagten Schutzfaktorgens in der Pflanze gewährleisten.

7. Verfahren gemäss Anspruch 1, **dadurch gekennzeichnet**, dass es sich bei besagter, für einen Schutzfaktor kodierenden DNA-Sequenz, um eine natürlich vorkommende DNA Sequenz handelt.

8. Verfahren gemäss Anspruch 1, **dadurch gekennzeichnet**, dass besagte DNA-Sequenz einen Schutzfaktor kodiert, der sich von einem natürlich vorkommenden Schutzfaktor unterscheidet, der aber noch im wesentlichen dieselbe Schutzfunktion erfüllt.

9. Verfahren gemäss einem der Ansprüche 7 oder 8, **dadurch gekennzeichnet**, dass es sich bei besagtem Schutzfaktor um eine antivirale Substanz handelt.

10. Verfahren gemäss einem der Ansprüche 7 oder 8, **dadurch gekennzeichnet**, dass es sich bei besagtem Schutzfaktor um eine zelltoxische Substanz handelt.

11. Verfahren gemäss Anspruch 9, **dadurch gekennzeichnet**, dass es sich bei besagter antiviraler Substanz um einen Antivirus-Antikörper, ein P_{R}-Protein, einen virus-spezifischen Proteaseinhibitor, eine Protease, einen virusspezifischen Polymerase-Inhibitor oder ein Interferon-ähnliches Protein handelt.

12. Verfahren gemäss Anspruch 10, **dadurch gekennzeichnet**, dass es sich bei besagter zelltoxischer Substanz um ein Toxin, eine aktive Untereinheit eines Toxins, ein P_{R}-Protein oder ein Protein handelt, welches natürlicherweise eine hypersensitive Reaktion auslöst.

13. Verfahren gemäss Anspruch 10, **dadurch gekennzeichnet**, dass es sich bei besagter zelltoxischer Substanz um ein Ribosomen-inaktivierendes Protein handelt.

14. Rekombinantes DNA Molekül, das in der Lage ist eine virusinduzierte Genexpression auf Pflanzen zu übertragen und das die folgenden Bestandteile aufweist:
(a) eine cis-aktive virusspezifische Kontrolregion, die über einen auf der Transkriptions-oder Posttranskriptionsebene wirksamen Transaktivierungsmechanismus reguliert wird; und
(b) in funktioneller Verknüpfung damit eine DNA Sequenz, welche einen antiviral oder zelltoxisch wirkenden Schutzfaktor kodiert, **der nicht identisch ist mit dem Hüllprotein des Alfalfa Mosaik Virus,** wobei besagte DNA Sequenz gegebenenfalls zusätzlich mit in der pflanzlichen Zelle wirksamen Expressionssignalen operabel verknüpft wird.

15. Rekombinantes DNA-Molekül gemäss Anspruch 14, **dadurch gekennzeichnet**, dass besagte, einen Schutzfaktor kodierende DNA-Sequenz ausschliesslich aus genomischer DNA, aus cDNA oder aus synthetischer DNA oder aber aus einem Gemisch besagter DNA's besteht.

16. Rekombinantes DNA-Molekül gemäss Anspruch 14, **dadurch gekennzeichnet**, dass besagte, einen induzierbaren Schutzfaktor-kodierende DNA-Sequenz in operabler Weise mit Expressionssignalen verknüpft ist, die in pflanzlichen Zellen aktiv sind.

17. Rekombinantes DNA-Molekül gemäss Anspruch 16, **dadurch gekennzeichnet**, dass es sich bei besagten Expressionssignalen um viruseigene Signale handelt.

18. Rekombinantes DNA-Molekül gemäss Anspruch 14, **dadurch gekennzeichnet**, dass es besagte virusspezifische Kontrollregion den ORF VII und die intergenomische Region zwischen ORF VII und ORF I des Cauliflower Mosaik-Virus umfasst.

19. Rekombinantes DNA-Molekül gemäss Anspruch 18, **dadurch gekennzeichnet**, dass es die komplette CaMV 35S Enhancer/Promoter-Region, die Leadersequenz, den Leserahmen ORF VII, die kleine intergenomische Region, 16 Kodons des Leserahmens ORF I, 17 Kodons von Linker- und Spacersequenzen, die für einen Schutzfaktor kodierende Region, Spacersequenzen, eine CaMV Polyadenylierungsstelle sowie Sequenzen des Plasmids pUC18 enthält.

20. Rekombinantes DNA-Molekül gemäss Anspruch 14, **dadurch gekennzeichnet**, dass es zwischen dem 19 S Promotor und dem CaMV Transkriptionsterminator den Leseraster eines Schutzfaktor-kodierenden Strukturgens enthält.

21. Verfahren zur Herstellung eines rekombinanten DNA Moleküls gemäss Anspruch 14, das durch die folgenden Verfahrensschritte gekennzeichnet ist:
(a) Isolierung und Klonierung einer DNA Sequenz, welche ein antiviral oder zelltoxisch wirkenden Schutzfaktor kodiert;
(b) funktionelle Verknüpfung besagter Schutzfaktor-kodierender DNA Sequenz mit einer cis-aktiven virusspezifischen Kontrolregion, die über einen auf der Transkriptions- oder Posttranskriptions bzw. -translationsebene wirksamen Transaktivierungsmechanismus reguliert wird, in einer Weise, dass die Induktion der Expression der Schutzfaktor-kodierenden DNA bei einer beginnenden Virusinfektion gewährleistet ist; und
(c) gegebenenfalls Einspleissen der hybriden Genkonstruktion gemäss Schritt (b) in operabler Weise zwischen in pflanzlichen Zellen aktiven Expressionssignalen.

22. Verfahren gemäss Anspruch 21, **dadurch gekennzeichnet**, dass die funktionelle Verknüpfung zwischen Schutzfaktor kodierender DNA-Sequenz und virusspezifischer Kontrollregion durch Austausch viraler, kodierender DNA-Sequenzen erfolgt, die selbst durch besagte virusspezifische Kontrollregion kontrolliert werden.

23. Verfahren gemäss Anspruch 21, **dadurch gekennzeichnet**, dass es sich bei besagten virusspezifischen Kontrollsequenzen um CaMV-Kontrollsequenzen handelt.

24. Verfahren gemäss Anspruch 21, **dadurch gekennzeichnet**, dass es sich bei besagten virusspezifischen Kontrollsequenzen um BMV-Kontrollsequenzen handelt.

25. Verfahren gemäss Anspruch 23, **dadurch gekennzeichnet**, dass besagte funktionelle Verknüpfung durch Austausch eines kodierenden DNA-Abschnittes des ORF1 auf dem CaMV-Genom gegen eine Schutzfaktor kodierende DNA Sequenz erfolgt.

26. Verfahren gemäss Anspruch 23, **dadurch gekennzeichnet**, dass besagte funktionelle Verknüpfung durch Austausch eines kodierenden DNA-Abschnittes des ORFVI auf dem CaMV-Genom gegen eine Schutzfaktor kodierende DNA Sequenz erfolgt.

27. Verfahren gemäss Anspruch 21, **dadurch gekennzeichnet**, dass besagte funktionelle Verknüpfung durch Austausch eines Hüllprotein-kodierenden cDNA-Abschnittes, der komplementär ist zu der entsprechenden Region auf der RNA3 von BMV, gegen eine Schutzfaktor kodierende DNA Sequenz erfolgt.

28. Verfahren gemäss Anspruch 21, **dadurch gekennzeichnet**, dass es sich bei besagten Expressionssignalen um Promotor- und Terminationssequenzen sowie um weitere regulatorische Sequenzen der 3'- und 5'-nichttranslatierten Region handelt, die in pflanzlichen Zellen aktiv sind.

29. Ein DNA-Transfer-Vektor, **dadurch gekennzeichnet**, dass er ein rekombinantes DNA-Molekül gemäss einem der Ansprüche 14 bis 20 enthält.

30. Ein DNA-Expressions-Vektor, **dadurch gekennzeichnet**, dass er ein rekombinantes DNA-Molekül gemäss einem der Ansprüche 14 bis 20 enthält.

31. Eine Wirtszelle, **dadurch gekennzeichnet**, dass sie
a) einen DNA-Transfer-Vektor gemäss Anspruch 29 enthält und
b) einen DNA-Expressions-Vektor gemäss Anspruch 30 enthält.

32. Eine Wirtszelle gemäss Anspruch 31, **dadurch gekennzeichnet**, dass es sich dabei um einen Mikroorganismus handelt.

33. Eine Wirtszelle gemäss Anspruch 31, **dadurch gekennzeichnet**, dass es sich dabei um eine pflanzliche Zelle handelt.

34. Virusresistente transgene Pflanze deren Zellen gesamthaft oder aber teilweise mit einem rekombinanten DNA Molekül gemäss einem der Ansprüche 14 bis 20 transformiert sind sowie die transgenen Samen besagter transgener Pflanze, **die ein rekombinantes DNA-Molekül gemäss einem der Ansprüche 14-20 enthalten.**

35. Eine pflanzliche Zelle, **dadurch gekennzeichnet**, dass sie mit einem rekombinanten DNA-Molekül gemäss einem der Ansprüche 14 bis 20 transformiert ist.

36. Eine pflanzliche Zelle gemäss Anspruch 35, **dadurch gekennzeichnet**, dass sie ein Bestandteil einer ganzen Pflanze ist.

37. Nachkommen einer virusresistenten, transgenen Pflanze gemäss Anspruch 34, **dadurch gekennzeichnet**, dass besagte Nachkommen aus pflanzlichen Zellen regeneriert worden sind, welche mit einem rekombinanten DNA-Molekül gemäss einem der Ansprüche 14 bis 20 transformiert sind und dass besagte Nachkommenschaft Mutanten und Varianten miteinschliesst, **die ein rekombinantes DNA-Molekül gemäss einem der Ansprüche 14 bis 20 enthalten.**

38. Lebensfähige Teile einer transgenen Pflanze gemäss einem der Ansprüche 34 oder 37 **sofern sie gesamthaft oder aber teilweise mit einem rekombinanten DNA Molekül** **gemäss einem der Ansprüche 14 bis 20 transformiert sind, dadurch gekennzeichnet**, dass es sich um Protoplasten, Zellen, Zellaggregate, Kallus, Samen, Pollen, Eizellen, Zygoten, oder Embryonen handelt.

39. Kreuzungs- und Fusionsprodukte mit dem in den Ansprüchen 33 bis 38 definierten Pflanzenmaterial, **welches noch ein rekombinantes DNA-Molekül gemäss einem der Ansprüche 14-20 enthält.**

40. Verfahren zur Herstellung virusresistenter, transgener Pflanzen, **dadurch gekennzeichnet**, dass man eine Pflanze oder lebensfähige Teile davon mit einem rekombinanten DNA Molekül gemäss einem der Ansprüche 14 bis 20 transformiert.

41. Verwendung eines rekombinanten DNA-Moleküls gemäss einem der Ansprüche 14 bis 20, zur "Immunisierung" von Pflanzen gegen einen unerwünschten Virusbefall, **dadurch gekennzeichnet**, dass man besagtes rekombinantes DNA Molekül in eine Pflanze einbringt.

## Claims

1. A process for the protection of plants against a virus infection and its sequelae, based on virus-intrinsic control mechanisms, **characterised in that**
(a) a DNA sequence coding for a protection factor having an antiviral or cell-toxic effect is operably linked with a cis-active virus-specific control region regulated by means of a transactivation mechanism acting at the transcription or post-transcription or post-translation level, and, where applicable, with expression signals that are active in plant cells; and
(b) the chimeric construct described in (a) is introduced into the plant to be protected, so that, after infection of the plant with viral DNA, a virus-induced expression of the protection factor gene occurs.

2. A process according to claim 1, **characterised in that** the virus-specific induction of the expression of the DNA sequence coding for a protection factor is effected by means of a transactivation of transcription.

3. A process according to claim 1, **characterised in that** the virus-specific induction of the expression of the DNA sequence coding for a protection factor occurs at the post-transcriptional level.

4. A process according to claim 3, **characterised in that** the virus-specific induction of the expression of the DNA sequence coding for a protection factor is effected by means of
a) a control of the mRNA elongation,
b) a control of mRNA transport from the nucleus into the cytoplasm,
c) a control of mRNA splicing,
d) a control of mRNA polyadenylation,
e) a control of the initiation of translation or
f) a differentiated use of different ORFs on a polycistronic RNA.

5. A process according to claim 1, **characterised in that** the virus-specific induction of the expression of the DNA sequence coding for a protection factor is effected by post-translational modification of the protein product.

6. A process according to claim 1, **characterised in that** the inducible DNA sequence coding for a protection factor is operably linked with expression signals that are active in plant cells and that ensure the expression of said protection factor gene in the plant.

7. A process according to claim 1, **characterised in that** said DNA sequence coding for a protection factor is a naturally occurring DNA sequence.

8. A process according to claim 1, **characterised in that** said DNA sequence codes for a protection factor that differs from a naturally occurring protection factor but still fulfils substantially the same protective function.

9. A process according to either claim 7 or claim 8, **characterised in that** said protection factor is an antiviral substance.

10. A process according to either claim 7 or claim 8, **characterised in that** said protection factor is a cell-toxic substance.

11. A process according to claim 9, **characterised in that** said antiviral substance is an antivirus-antibody, a P_{R}-protein, a virus-specific protease inhibitor, a protease, a virus-specific polymerase inhibitor or an interferon-like protein.

12. A process according to claim 10, **characterised in that** said cell-toxic substance is a toxin, an active subunit of a toxin, a P_{R}-protein or a protein that naturally triggers a hypersensitive reaction.

13. A process according to claim 10, **characterised in that** said cell-toxic substance is a ribosome-inactivating protein.

14. A recombinant DNA molecule that is capable of conferring a virus-induced gene expression upon plants and that has the following constituents:
(a) a cis-active virus-specific control region regulated by means of a transactivation mechanism acting at the transcription or post-transcription level; and
(b) functionally linked therewith, a DNA sequence coding for a protection factor having an antiviral or cell-toxic effect, **which protection factor is not identical with the coat protein of alfalfa mosaic virus,** where applicable said DNA sequence being, in addition, operably linked with expression signals that are active in plant cells.

15. A recombinant DNA molecule according to claim 14, **characterised in that** said DNA sequence coding for a protection factor consists exclusively of genomic DNA, of cDNA or of synthetic DNA, or alternatively of a mixture of said DNAs.

16. A recombinant DNA molecule according to claim 14, **characterised in that** said DNA sequence coding for an inducible protection factor is operably linked with expression signals that are active in plant cells.

17. A recombinant DNA molecule according to claim 16, **characterised in that** said expression signals are virus-intrinsic signals.

18. A recombinant DNA molecule according to claim 14, **characterised in that** said virus-specific control region comprises ORF VII and the intergenomic region between ORF VII and ORF I of cauliflower mosaic virus.

19. A recombinant DNA molecule according to claim 18, **characterised in that** it contains the complete CaMV 35S enhancer/promoter region, the leader sequence, the reading frame ORF VII, the small intergenomic region, 16 codons of the reading frame ORF I, 17 codons of linker and spacer sequences, the region coding for a protection factor, spacer sequences, a CaMV polyadenylation site and sequences of the plasmid pUC18.

20. A recombinant DNA molecule according to claim 14, **characterised in that** it contains, between the 19S promoter and the CaMV transcription terminator, the reading frame of a structural gene coding for a protection factor.

21. A process for the preparation of a recombinant DNA molecule according to claim 14, which process is characterised by the following steps:
a) isolation and cloning of a DNA sequence coding for a protection factor having an antiviral or cell-toxic effect;
b) functional linking of said DNA sequence coding for a protection factor with a cis-active virus-specific control region regulated by means of a transactivation mechanism acting at the transcription or post-transcription or post-translation level, in such a manner that the induction of the expression of the DNA coding for the protection factor is ensured when a virus infection begins; and
c) where applicable, splicing in of the hybrid gene construct described in step b) in operable manner between expression signals that are active in plant cells.

22. A process according to claim 21, **characterised in that** the functional linking between the DNA sequence coding for a protection factor and the virus-specific control region is effected by replacement of viral coding DNA sequences that are themselves controlled by said virus-specific control region.

23. A process according to claim 21, **characterised in that** said virus-specific control sequences are CaMV control sequences.

24. A process according to claim 21, **characterised in that** said virus-specific control sequences are BMV control sequences.

25. A process according to claim 23, **characterised in that** said functional linking is effected by replacing a coding DNA region of ORF I on the CaMV genome by a DNA sequence coding for a protection factor.

26. A process according to claim 23, **characterised in that** said functional linking is effected by replacing a coding DNA region of ORF VI on the CaMV genome by a DNA sequence coding for a protection factor.

27. A process according to claim 21, **characterised in that** said functional linking is effected by replacing a cDNA region coding for a coat protein that is complementary to the corresponding region on the RNA3 of BMV by a DNA sequence coding for a protection factor.

28. A process according to claim 21, **characterised in that** said expression signals are promoter and termination sequences and other regulatory sequences of the 3'- and 5'-non-translated region that are active in plant cells.

29. A DNA transfer vector, **characterised in that** it contains a recombinant DNA molecule according to any one of claims 14 to 20.

30. A DNA expression vector, **characterised in that** it contains a recombinant DNA molecule according to any one of claims 14 to 20.

31. A host cell, **characterised in that** it contains
a) a DNA transfer vector according to claim 29 and
b) a DNA expression vector according to claim 30.

32. A host cell according to claim 31, **characterised in that** it is a microorganism.

33. A host cell according to claim 31, **characterised in that** it is a plant cell.

34. A virus-resistant transgenic plant the cells of which have been entirely or partially transformed with a recombinant DNA molecule according to any one of claims 14 to 20 and the transgenic seeds of said transgenic plant, **which seeds contain a recombinant DNA molecule according to any one of claims 14 to 20.**

35. A plant cell, **characterised in that** it has been transformed with a recombinant DNA molecule according to any one of claims 14 to 20.

36. A plant cell according to claim 35, **characterised in that** it is a component part of an entire plant.

37. Descendants of a virus-resistant transgenic plant according to claim 34, **characterised in that** said descendants have been regenerated from plant cells that have been transformed with a recombinant DNA molecule according to any one of claims 14 to 20 and that said descendants include mutants and variants **that contain a recombinant DNA molecule according to any one of claims 14 to 20.**

38. Viable parts of a transgenic plant according to either claim 34 or claim 37 **provided they have been entirely or partially transformed by a recombinant DNA molecule according to any one of claims 14 to 20, characterised in that** they are protoplasts, cells, cell aggregates, callus, seeds, pollen, ova, zygotes or embryos.

39. Hybridization and fusion products with the plant material defined in claims 33 to 38 **that still contains a recombinant DNA molecule according to any one of claims 14 to 20.**

40. A process for the production of virus-resistant transgenic plants, **characterised in that** a plant or viable parts thereof is (are) transformed with a recombinant DNA molecule according to any one of claims 14 to 20.

41. The use of a recombinant DNA molecule according to any one of claims 14 to 20 for the "immunisation" of plants against undesired virus attack, **characterised in that** said recombinant DNA molecule is introduced into a plant.

## Revendications

1. Procédé pour la protection de plantes contre une infection virale et ses suites, sur la base de mécanismes régulateurs propres au virus, **caractérisé en ce que**
(a) une séquence d'ADN qui code pour un facteur de protection à action antivirale ou cytotoxique est fonctionnellement liée à une région régulatrice spécifique de virus, active en *cis*, qui est régulée par un mécanisme d'activation en *trans* actif au niveau de la transcription ou post-transcription ou post-traduction, ainsi qu'éventuellement à des signaux d'expression actifs dans la cellule végétale ; et
(b) le produit de construction chimère selon (a) est introduit dans la plante à protéger, de sorte qu'après infection de la plante avec de l'ADN viral, on aboutit à une expression, induite par un virus, du gène du facteur de protection.

2. Procédé selon la revendication 1, **caractérisé en ce que** l'induction, spécifique de virus, de l'expression de la séquence d'ADN codant pour un facteur de protection s'effectue par l'intermédiaire d'une activation en *trans* de la transcription.

3. Procédé selon la revendication 1, **caractérisé en ce que** l'induction, spécifique de virus, de l'expression de la séquence d'ADN codant pour un facteur de protection s'effectue au niveau post-transcriptionnel.

4. Procédé selon la revendication 1, **caractérisé en ce que** l'induction, spécifique de virus, de l'expression de la séquence d'ADN codant pour un facteur de protection s'effectue par l'intermédiaire
a) d'une régulation de l'extension d'ARNm,
b) d'une régulation du transport d'ARNm du noyau dans le cytoplasme,
c) d'une régulation de l'épissage d'ARNm,
d) d'une régulation de la polyadénylation d'ARNm,
e) d'une régulation de l'initiation de traduction ou
f) d'une utilisation différenciée de divers ORF (cadres de lecture ouverts) sur un ARN polycistronique.

5. Procédé selon la revendication 1, **caractérisé en ce que** l'induction, spécifique de virus, de l'expression de la séquence d'ADN codant pour un facteur de protection s'effectue par modification post-traductionnelle du produit protéique.

6. Procédé selon la revendication 1, **caractérisé en ce qu**'on raccorde la séquence d'ADN inductible, codant pour un facteur de protection, de façon fonctionnelle avec des signaux d'expression qui sont actifs dans la cellule végétale et qui garantissent l'expression dudit gène de facteur de protection dans la plante.

7. Procédé selon la revendication 1, caractérisé par le fait qu'en ce qui concerne ladite séquence d'ADN codant pour un facteur de protection, il s'agit d'une séquence d'ADN existant dans la nature.

8. Procédé selon la revendication 1, **caractérisé en ce que** ladite séquence d'ADN code pour un facteur de protection qui se distingue d'un facteur de protection existant dans la nature, mais qui remplit encore pratiquement la même fonction protectrice.

9. Procédé selon la revendication 7 ou 8, **caractérisé en ce que** ledit facteur de protection est une substance antivirale.

10. Procédé selon la revendication 7 ou 8, **caractérisé en ce que** ledit facteur de protection est une substance cytotoxique.

11. Procédé selon la revendication 9, **caractérisé en ce que** ladite substance antivirale est un anticorps anti-virus, une protéine P_{R}, un inhibiteur de protéase spécifique de virus, une protéase, un inhibiteur de polymérase spécifique de virus ou une protéine de type interféron.

12. Procédé selon la revendication 10, **caractérisé en ce que** ladite substance cytotoxique est une toxine, une sous-unité active d'une toxine, une protéine P_{R} ou une protéine qui déclenche naturellement une réaction d'hypersensibilité.

13. Procédé selon la revendication 10, **caractérisé en ce que** ladite substance cytotoxique est une protéine inactivant les ribosomes.

14. Molécule d'ADN recombinant, qui est capable de transférer à des plantes une expression de gène induite par un virus et qui comporte les composants suivants :
(a) une région régulatrice spécifique de virus, active en *cis*, qui est régulée par un mécanisme d'activation en *trans* actif au niveau transcriptionnel ou post-transcriptionnel ; et
(b) en liaison fonctionnelle avec celle-ci, une séquence d'ADN qui code pour un facteur de protection à action antivirale ou cytotoxique, **qui n'est pas identique à la protéine d'enveloppe du virus de la mosaïque de la luzerne,** ladite séquence d'ADN étant éventuellement en outre fonctionnellement raccordée à des signaux d'expression actifs dans la cellule végétale.

15. Molécule d'ADN recombinant selon la revendication 14, **caractérisée en ce que** ladite séquence d'ADN codant pour un facteur de protection consiste exclusivement en ADN génomique, en ADNc ou en ADN synthétique ou bien en un mélange desdits ADN.

16. Molécule d'ADN recombinant selon la revendication 14, **caractérisée en ce que** ladite séquence d'ADN codant pour un facteur de protection inductible est raccordée de façon fonctionnelle à des signaux d'expression qui sont actifs dans des cellules végétales.

17. Molécule d'ADN recombinant selon la revendication 16, **caractérisée en ce que** lesdits signaux d'expression sont des signaux propres à un virus.

18. Molécule d'ADN recombinant selon la revendication 14, **caractérisée en ce que** ladite région régulatrice spécifique de virus comprend l'ORF VII et la région intergénomique entre ORF VII et ORF I du virus de la mosaïque du chou-fleur.

19. Molécule d'ADN recombinant selon la revendication 18, **caractérisée en ce qu**'elle contient la région complète d'activateur/promoteur de CaMV 35S, la séquence de tête, le cadre de lecture ORF VII, la courte région intergénomique, 16 codons du cadre de lecture ORF I, 17 codons de séquences de lieur et d'espaceur, la région codant pour un facteur de protection, des séquences d'espaceur, un site de polyadénylation de CaMV, ainsi que des séquences du plasmide pUC18.

20. Molécule d'ADN recombinant selon la revendication 14, **caractérisée en ce qu**'entre le promoteur 19 S et le terminateur de transcription de CaMV, elle contient le cadre de lecture d'un gène structural codant pour un facteur de protection.

21. Procédé pour la production d'une molécule d'ADN recombinant selon la revendication 14, qui est caractérisé par les étapes de processus suivantes :
(a) isolement et clonage d'une séquence d'ADN qui code pour un facteur de protection antiviral ou cytotoxique ;
(b) raccordement fonctionnel de ladite séquence d'ADN codant pour un facteur de protection, avec une région régulatrice spécifique de virus, active en *cis*, qui est régulée par un mécanisme d'activation en *trans* actif au niveau de la transcription ou post-transcription ou post-traduction, d'une façon qui garantit l'induction de l'expression de l'ADN codant pour un facteur de protection, lors d'une infection virale débutante ; et
(c) épissage éventuel du produit de construction hybride selon l'étape (b), de façon fonctionnelle, entre des signaux d'expression actifs dans des cellules végétales.

22. Procédé selon la revendication 21, **caractérisé en ce que** le raccordement fonctionnel entre séquence d'ADN codant pour un facteur de protection et région régulatrice spécifique de virus s'effectue par échange de séquences d'ADN virales codantes, qui sont elles-mêmes régulées par ladite région régulatrice spécifique de virus.

23. Procédé selon la revendication 21, caractérisé par le fait qu'en ce qui concerne lesdites séquences régulatrices spécifiques de virus, il s'agit de séquences régulatrices de CaMV.

24. Procédé selon la revendication 21, caractérisé par le fait qu'en ce qui concerne lesdites séquences régulatrices spécifiques de virus, il s'agit de séquences régulatrices de BMV.

25. Procédé selon la revendication 23, **caractérisé en ce que** ledit raccordement fonctionnel s'effectue par échange d'un segment d'ADN codant de l'ORF I sur le génome de CaMV contre une séquence d'ADN codant pour un facteur de protection.

26. Procédé selon la revendication 23, **caractérisé en ce que** ledit raccordement fonctionnel s'effectue par échange d'un segment d'ADN codant de l'ORF VI sur le génome de CaMV contre une séquence d'ADN codant pour un facteur de protection.

27. Procédé selon la revendication 21, **caractérisé en ce que** ledit raccordement fonctionnel s'effectue par échange d'un segment d'ADN codant pour une protéine d'enveloppe, qui est complémentaire de la région correspondante sur l'ARN3 de BMV, contre une séquence d'ADN codant pour un facteur de protection.

28. Procédé selon la revendication 1, caractérisé par le fait qu'en ce qui concerne lesdits signaux d'expression, il s'agit de séquences de promoteur et de terminaison ainsi que d'autres séquences régulatrices de la région non traduite en 3' et en 5', qui sont actives dans des cellules végétales.

29. Vecteur de transfert d'ADN, **caractérisé en ce qu**'il contient une molécule d'ADN recombinant selon l'une quelconque des revendications 14 à 20.

30. Vecteur d'expression d'ADN, **caractérisé en ce qu**'il contient une molécule d'ADN recombinant selon l'une quelconque des revendications 14 à 20.

31. Cellule hôte, **caractérisée en ce qu**'elle contient
a) un vecteur de transfert d'ADN selon la revendication 29 et
b) un vecteur d'expression d'ADN selon la revendication 30.

32. Cellule hôte selon la revendication 31, **caractérisée en ce qu**'il s'agit d'un micro-organisme.

33. Cellule hôte selon la revendication 31, **caractérisée en ce qu**'il s'agit d'une cellule végétale.

34. Plante transgénique résistante à un virus, dont les cellules sont en totalité ou bien en partie transformées par une molécule d'ADN recombinant selon l'une quelconque des revendications 14 à 20, ainsi que les graines transgéniques de ladite plante transgénique **qui contiennent une molécule d'ADN recombinant selon l'une quelconque des revendications 14 à 20.**

35. Cellule végétale, **caractérisée en ce qu**'elle est transformée par une molécule d'ADN recombinant selon l'une quelconque des revendications 14 à 20.

36. Cellule végétale selon la revendication 35, **caractérisée en ce qu**'elle est un composant d'une plante entière.

37. Descendance d'une plante transgénique résistante à un virus, selon la revendication 34, **caractérisée en ce que** ladite descendance a été régénérée à partir de cellules végétales qui sont transformées par une molécule d'ADN recombinant selon l'une quelconque des revendications 14 à 20 et en ce que ladite descendance comprend des mutants et variants **qui contiennent une molécule d'ADN recombinant selon l'une quelconque des revendications 14 à 20.**

38. Parties viables d'une plante transgénique selon l'une quelconque des revendications 34 et 37, **dans la mesure où elles sont en totalité ou bien en partie transformées par une molécule d'ADN recombinant selon l'une quelconque des revendications 14 à 20, caractérisées en ce qu**'il s'agit de protoplastes, cellules, agrégats de cellules, cals, graines, pollen, ovules, zygotes ou embryons.

39. Produits de croisement et de fusion avec le matériel végétal défini dans les revendications 33 à 38, **qui contient encore une molécule d'ADN recombinant selon l'une quelconque des revendications 14 à 20.**

40. Procédé pour la production de plantes transgéniques résistantes à des virus, **caractérisé en ce qu**'on transforme une plante ou des parties viables de celle-ci avec une molécule d'ADN recombinant selon l'une quelconque des revendications 14 à 20.

41. Utilisation d'une molécule d'ADN recombinant selon l'une quelconque des revendications 14 à 20, pour l' "immunisation" de plantes contre une attaque par un virus indésirable, **caractérisée en ce qu**'on introduit dans une plante ladite molécule d'ADN recombinant.
